# EUROPEAN PATENT APPLICATION

(11) **EP 4 813 401 A1**
(43) Date of publication of application: **30.09.2026**
(21) Application number: 24895854.8
(22) Date of filing: 04.09.2024
(51) Int. Cl.: A61K 35/76, A61K 39/395, A61K 39/00, A61P 35/00, A61K 48/00

(54) **METHOD FOR TREATING TUMOR WITH COMBINATION OF RECOMBINANT ONCOLYTIC VIRUS AND MACROMOLECULAR ANTIBODY-BASED ANTI-CANCER DRUG**

(30) Priority: 27.11.2023 CN 202311584736
(71) Applicant: Joint Biosciences (SH) Ltd., Pudong New Area Shanghai 201318 (CN)
(72) Inventor: ZHOU, Guoqing, Shanghai 201318 (CN); MA, Liang, Shanghai 201318 (CN); TIAN, Ting, Shanghai 201318 (CN); CHENG, Longxin, Shanghai 201318 (CN); MA, Qibin, Shanghai 201318 (CN); MAO, Liying, Shanghai 201318 (CN); ZHANG, Fan, Shanghai 201318 (CN)
(74) Representative: Dr. Gassner & Partner mbB
(86) International application number: PCT/CN2024/116826
(87) International publication number: WO 2025/112755

(57) **Abstract**

The present invention relates to the field of biopharmaceutical technology, and particularly, to a method for treating a tumor with a combination of a recombinant oncolytic virus and a macromolecular antibody-based anti-cancer drug. The method includes: using the recombinant oncolytic virus and the macromolecular antibody-based anti-cancer drug in combination to treat the tumor, wherein the macromolecular antibody-based anti-cancer drug includes, but is not limited to, macromolecular antibody-based anti-cancer drugs targeting HER2, EGFR, 5T4, B7-H3, TROP2, BCMA, VEGFR-2, SLAMF7, CD3, CD19, CD20, CD22, CD30, CD33, CD38, CD52, CD79b, GD2, VEGF, CTLA4, Claudin18.2, PD-1, PD-L1, and the like, i.e., monoclonal antibody-based drugs, polyclonal antibody-based drugs, antibody-drug conjugates and the like. The method can achieve a therapeutic effect comparable or superior to those of the monotherapies, or even a synergistic effect.

## Description

### TECHNICAL FIELD

The present application relates to the field of biopharmaceutical technology, and particularly, to a method for treating a tumor with a combination of a recombinant oncolytic virus and a macromolecular antibody-based anti-cancer drug.

### BACKGROUND ART

Oncolytic viruses are a class of replication-competent tumoricidal viruses that currently have been widely recognized as an important branch of tumor immunotherapy. Oncolytic viruses are capable of specifically targeting and infecting tumor cells, for example, by utilizing the inactivation or defects of genes that inhibit oncolytic viruses in tumor cells to achieve selective infection of tumor cells. Following infection of tumor cells with oncolytic viruses, the oncolytic viruses will replicate extensively within the tumor cells and ultimately lyse the tumor cells, resulting in tumor cell death. In addition, oncolytic viruses can also provide the essential immunostimulatory signals required to elicit host's intrinsic anti-cancer responses, thereby recruiting additional immune cells to further eliminate residual tumor cells.

Although oncolytic viruses have shown promising prospects in tumor immunotherapy, wild-type oncolytic viruses often cause tissue and organ damage as well as functional disorders in the hosts. Moreover, the use of wild-type viruses to infect tumor cells is associated with significant pathogenic risks. Therefore, to further advance the clinical application of oncolytic viruses, it is necessary to modify wild-type oncolytic viruses to obtain attenuated oncolytic viruses. The clinical use of such attenuated oncolytic viruses can reduce pathogenic risks and improve the safety profile of oncolytic viruses.

However, during the modification of oncolytic viruses, if only wild-type oncolytic viruses are subjected to random genetic modifications, although the toxicity may be reduced, the resulting modified oncolytic viruses may exhibit poor therapeutic efficacy, or even fail to be packaged, thereby hindering the clinical application of oncolytic viruses.

With the development of modern molecular biology and the application of advanced technologies such as computer-aided drug design, structural biology, and combinatorial chemistry, the development of macromolecular drugs has accelerated significantly. Biomacromolecular drugs, including peptides, proteins, antibodies, glycans, and nucleic acids, are widely used in the treatment of major diseases such as tumors, AIDS, cardiovascular and cerebrovascular diseases, and hepatitis, and are recognized as one of the most promising areas in drug research and development in the 21st century. Currently, significant challenges and obstacles in the use of biomacromolecular drugs remain to be urgently addressed. For example, such drugs often exhibit poor membrane permeability, strong immunogenicity, poor penetration into solid tumors, morphological complexity (including polymorphism, multiple conformations, and multiscale characteristics), as well as difficulties in separation and purification and low stability. As a result, the clinical application of macromolecular drugs is limited to a certain extent.

### SUMMARY

In order to further improve the therapeutic effect on tumor cells, the present application provides a method for treating a tumor with a combination of a recombinant oncolytic virus and a macromolecular antibody-based anti-cancer drug.

Oncolytic viruses are a class of replication-competent tumoricidal viruses that currently have been widely recognized as an important branch of tumor immunotherapy. Oncolytic viruses are capable of specifically targeting and infecting tumor cells, for example, by utilizing the inactivation or defects of genes that inhibit oncolytic viruses in tumor cells to achieve selective infection of tumor cells. Following infection of tumor cells with oncolytic viruses, the oncolytic viruses will replicate extensively within the tumor cells and ultimately lyse the tumor cells, resulting in tumor cell death. Meanwhile, oncolytic viruses can also provide the essential immunostimulatory signals required to enhance host's intrinsic anti-cancer responses, thereby recruiting additional immune cells to further eliminate residual tumor cells. Therefore, oncolytic viruses have the ability to disrupt the tumor tissue microenvironment, turning "cold tumors" into "hot tumors".

The present application is based on the "Mark + Kill" principle. A recombinant oncolytic viral vaccine is engineered to express tumor antigen targets, thereby not only targeting tumor cells but also transforming tumors that originally lacked targets and were thus untreatable by antibodies into treatable tumors with targets (referred to as "Mark"). The recombinant oncolytic viral vaccine is combined with monoclonal antibodies, bispecific antibodies, polyclonal antibodies, or antibody-drug conjugates (ADCs) that target the antigen targets. This combination therapy achieves or surpasses a therapeutic effect of a recombinant oncolytic virus monotherapy or a macromolecular antibody-based anti-cancer drug monotherapy, or even a synergistic effect (referred to as Kill).

Furthermore, to enhance the therapeutic effect, cytokines can be inserted and expressed in the oncolytic virus. Through the synergistic interaction of the three antitumor mechanisms-oncolytic viruses, cytokines, and macromolecular antibody-based anti-cancer drugs-a more potent anti-tumor effect can be achieved.

The present application provides a method for treating a tumor with a combination of a recombinant oncolytic virus and a macromolecular antibody-based anti-cancer drug, adopting the following technical solution:

A method for treating a tumor with a combination of a recombinant oncolytic virus and a macromolecular antibody-based anti-cancer drug, using the macromolecular antibody-based anti-cancer drug and the recombinant oncolytic virus in combination to treat the tumor.

The macromolecular antibody-based anti-cancer drug includes, but is not limited to, a macromolecular antibody-based anti-cancer drug targeting HER2, a macromolecular antibody-based anti-cancer drug targeting EGFR, a macromolecular antibody-based anti-cancer drug targeting PD-1, a macromolecular antibody-based anti-cancer drug targeting PD-L1, a macromolecular antibody-based anti-cancer drug targeting TROP2, a macromolecular antibody-based anti-cancer drug targeting BCMA, a macromolecular antibody-based anti-cancer drug targeting VEGFR-2, a macromolecular antibody-based anti-cancer drug targeting SLAMF7, a macromolecular antibody-based anti-cancer drug targeting CD3, a macromolecular antibody-based anti-cancer drug targeting CD19, a macromolecular antibody-based anti-cancer drug targeting CD20, a macromolecular antibody-based anti-cancer drug targeting CD22, a macromolecular antibody-based anti-cancer drug targeting CD30, a macromolecular antibody-based anti-cancer drug targeting CD33, a macromolecular antibody-based anti-cancer drug targeting CD38, a macromolecular antibody-based anti-cancer drug targeting CD52, a macromolecular antibody-based anti-cancer drug targeting CD79b, a macromolecular antibody-based anti-cancer drug targeting Met, a macromolecular antibody-based anti-cancer drug targeting GD2, a macromolecular antibody-based anti-cancer drug targeting VEGF, a macromolecular antibody-based anti-cancer drug targeting PDGFR-α, a macromolecular antibody-based anti-cancer drug targeting CTLA-4, a macromolecular antibody-based anti-cancer drug targeting RANKL, a macromolecular antibody-based anti-cancer drug targeting FRa, a macromolecular antibody-based anti-cancer drug targeting TF, a macromolecular antibody-based anti-cancer drug targeting IL6, a macromolecular antibody-based anti-cancer drug targeting GPRC5D, a macromolecular antibody-based anti-cancer drug targeting TNF-α, a macromolecular antibody-based anti-cancer drug targeting EPCAM, a macromolecular antibody-based anti-cancer drug targeting CD24, a macromolecular antibody-based anti-cancer drug targeting 5T4, a macromolecular antibody-based anti-cancer drug targeting B7-H3, a macromolecular antibody-based anti-cancer drug targeting GPC3, and a macromolecular antibody-based anti-cancer drug targeting Claudin 18.2.

The recombinant oncolytic virus includes an M protein, a G protein, an N protein, a P protein, and an L protein.

Compared with an amino acid sequence as set forth in SEQ ID NO 1, an site mutation of the M protein includes one or more of M51R, V221F, or S226R; alternatively, the site mutation of the M protein includes one or more of N32S, N49D, M51R, H54Y, V221F, V225I, or S226R; alternatively, the site mutation of the M protein includes one or more of N32S, N49D, M51R, H54Y, knocking out of nucleotide bases encoding leucine at position 111, V221F, V225I, or S226R; alternatively, the site mutation of the M protein includes one or more of N32S, N49D, M51R, H54Y, L111A, V221F, V225I, or S226R; alternatively, the site mutation of the M protein includes one or more of G21E, N32S, N49D, M51R, H54Y, V221F, V225I, or S226R; alternatively, the site mutation of the M protein includes one or more of G21E, N32S, M33A, N49D, M51R, H54Y, V221F, V225I, or S226R; alternatively, the site mutation of the M protein includes one or more of G21E, N32S, M33A, N49D, M51R, H54Y, A133T, V221F, V225I, or S226R; alternatively, the site mutation of the M protein includes one or more of N32S, M33A, N49D, M51R, H54Y, V221F, V225I, or S226R; alternatively, the site mutation of the M protein includes one or more of N32S, M33A, N49D, M51R, H54Y, A133T, V221F, V225I, or S226R; alternatively, the site mutation of the M protein includes one or more of N32S, N49D, M51R, H54Y, A133T, V221F, V225I, or S226R.

Compared with an amino acid sequence as set forth in SEQ ID NO 12, an site mutation of the G protein includes one or more of V53I, A141V, D172Y, K217E, D232G, V331A, V371E, G436D, T438S, F453L, T471I, or Y487H.

Compared with an amino acid sequence as set forth in SEQ ID NO 14, an site mutation of the N protein includes one or more of I14V, R155K, or S353N.

Compared with an amino acid sequence as set forth in SEQ ID NO 16, an site mutation of the P protein includes one or more of R50K, V76A, D99E, L126S, L140S, H151Y, I168M, K170E, Y189S, or N237D.

Compared with an amino acid sequence as set forth in SEQ ID NO 18, an site mutation of the L protein includes one or more of S87P or I487T.

In the present application, the M protein of a wild-type VSV Indiana MuddSummer strain includes an amino acid sequence as set forth in SEQ ID NO 1.

In a specific embodiment, the M protein includes an amino acid sequence as set forth in SEQ ID NO 2.

In a specific embodiment, the M protein includes an amino acid sequence as set forth in SEQ ID NO 3.

In a specific embodiment, the M protein includes an amino acid sequence as set forth in SEQ ID NO 4.

In a specific embodiment, the M protein includes an amino acid sequence as set forth in SEQ ID NO 5.

In a specific embodiment, the M protein includes an amino acid sequence as set forth in SEQ ID NO 6.

In a specific embodiment, the M protein includes an amino acid sequence as set forth in SEQ ID NO 7.

In a specific embodiment, the M protein includes an amino acid sequence as set forth in SEQ ID NO 8.

In a specific embodiment, the M protein includes an amino acid sequence as set forth in SEQ ID NO 9.

In a specific embodiment, the M protein includes an amino acid sequence as set forth in SEQ ID NO 10.

In a specific embodiment, the M protein includes an amino acid sequence as set forth in SEQ ID NO 11.

In the present application, the G protein of the wild-type VSV Indiana MuddSummer strain includes an amino acid sequence as set forth in SEQ ID NO 12.

In a specific embodiment, the G protein includes an amino acid sequence as set forth in SEQ ID NO 13.

In the present application, the N protein of the wild-type VSV Indiana MuddSummer strain includes an amino acid sequence as set forth in SEQ ID NO 14.

In a specific embodiment, the N protein includes an amino acid sequence as set forth in SEQ ID NO 15.

In the present application, the P protein of wild-type VSV Indiana MuddSummer strain includes an amino acid sequence as set forth in SEQ ID NO 16.

In a specific embodiment, the P protein includes an amino acid sequence as set forth in SEQ ID NO 17.

In the present application, the L protein of the wild-type VSV Indiana MuddSummer strain includes an amino acid sequence as set forth in SEQ ID NO 18.

In a specific embodiment, the L protein includes an amino acid sequence as set forth in SEQ ID NO 19.

In some specific embodiments, the recombinant oncolytic virus is obtained from site-directed mutagenesis based on a rhabdovirus.

In some specific embodiments, the recombinant oncolytic virus is obtained from site-directed mutagenesis based on a vesicular stomatitis virus (referred to as "VSV").

In some specific embodiments, the recombinant oncolytic virus is obtained from site-directed mutagenesis based on a VSV Indiana MuddSummer strain.

Furthermore, the antigen is one or more selected from a group consisting of: CD19, CD22, BCMA, MUC1, NY-ESO-1, MAGE A4, MET, Claudin 18.2, MSLN, EGFR, VEGFR2, HER2, TPBG, AFP, and MAGE-A10. Furthermore, the recombinant oncolytic virus further includes a cytokine encoded by an exogenous gene.

Furthermore, the cytokine is one or more selected from a group consisting of: GMCSF, IL-2, IL-12, IL-15, IL-18, TNF-α, and IFN-β.

The antigen sequence inserted in the recombinant oncolytic virus may be a full-length sequence or a partial specific sequence. Similarly, the target sequence inserted in the recombinant oncolytic virus may be a full-length sequence or a partial specific sequence. Similarly, the cytokine sequence inserted in the recombinant oncolytic virus may be a full-length sequence or a partial specific sequence.

The present application further provides a composition, which includes the recombinant oncolytic virus (an oncolytic viral vaccine) and the macromolecular antibody-based anti-cancer drug as described above.

In summary, the present application has the following beneficial effects:

The method for treating a tumor with a combination of a recombinant oncolytic virus and a macromolecular antibody-based anti-cancer drug provided in the present application exhibits a significant inhibitory effect on tumor growth. In addition, the test results of tumor treatment using a recombinant oncolytic virus directly combined with a macromolecular antibody-based anti-cancer drug are superior to those of a recombinant oncolytic virus monotherapy of tumor or a macromolecular antibody-based anti-cancer drug monotherapy of tumor. The test results of tumor treatment using a recombinant oncolytic virus inserted with and expressing an antigen or antigen fragment combined with a macromolecular antibody-based anti-cancer drug targeting the antigen or antigen fragment are superior to the test results of tumor treatment using a recombinant oncolytic virus directly combined with a macromolecular antibody-based anti-cancer drug or the test results of tumor treatment directly using a recombinant oncolytic virus inserted with and expressing an antigen or antigen fragment. Therefore, the method for treating a tumor with a combination of a recombinant oncolytic virus and a macromolecular antibody-based anti-cancer drug provided in the present application further effectively improves the therapeutic effects on tumor cells.

The method for treating a tumor with the combination of a recombinant oncolytic virus and a macromolecular antibody-based anti-cancer drug provided in the present application exhibits good inhibitory effects on the growth of tumor cells. It can be inferred that the method for treating a tumor with the combination of a recombinant oncolytic virus and a macromolecular antibody-based anti-cancer drug provided in the present application further exhibits favorable inhibitory effects on other cancer cells, proposing broad clinical application prospects.

### BRIEF DESCRIPTION OF THE DRAWINGS

FIG. 1 shows the results of an animal experiment in which a recombinant oncolytic virus and a macromolecular antibody-based anti-cancer drug, Trastuzumab, are used in combination to treat a tumor.
FIG. 2 shows the results of an animal experiment in which a recombinant oncolytic virus and a macromolecular antibody-based anti-cancer drug, Necitumumab, are used in combination to treat a tumor.
FIG. 3 shows the results of an animal experiment in which a recombinant oncolytic virus and a macromolecular antibody-based anti-cancer drug, Anti-5T4 antibody, are used in combination to treat a tumor.
FIG. 4 shows the results of an animal experiment in which a recombinant oncolytic virus and a macromolecular antibody-based anti-cancer drug, Anti-CD276 antibody, are used in combination to treat a tumor.
FIG. 5 shows the results of an animal experiment in which a recombinant oncolytic virus and a macromolecular antibody-based anti-cancer drug, Sacituzumab govitecan, are used in combination to treat a tumor.
FIG. 6 shows the results of an animal experiment in which a recombinant oncolytic virus and a macromolecular antibody-based anti-cancer drug, Belantamab Mafodotin, are used in combination to treat a tumor.
FIG. 7 shows the results of an animal experiment in which a recombinant oncolytic virus and a macromolecular antibody-based anti-cancer drug, Ramucirumab, are used in combination to treat a tumor.
FIG. 8 shows the results of an animal experiment in which a recombinant oncolytic virus and a macromolecular antibody-based anti-cancer drug, Elotuzumab, are used in combination to treat a tumor.
FIG. 9 shows the results of an animal experiment in which a recombinant oncolytic virus and a macromolecular antibody-based anti-cancer drug, Blinatumomab, are used in combination to treat a tumor.
FIG. 10 shows the results of an animal experiment in which a recombinant oncolytic virus and a macromolecular antibody-based anti-cancer drug, Loncastuximab tesirine, are used in combination to treat a tumor.
FIG. 11 shows the results of an animal experiment in which a recombinant oncolytic virus and a macromolecular antibody-based anti-cancer drug, Polatuzumab vedotin, are used in combination to treat a tumor.
FIG. 12 shows the results of an animal experiment in which a recombinant oncolytic virus and a macromolecular antibody-based anti-cancer drug, Anti-Glypican3 antibody, are used in combination to treat a tumor.
FIG. 13 shows the results of an animal experiment in which a recombinant oncolytic virus and a macromolecular antibody-based anti-cancer drug, Anti-Claudin18.2 antibody, are used in combination to treat a tumor.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### DETAILED DESCRIPTION

The implementation of the present application will be illustrated in the following specific examples, and other advantages and effects of the present application will be easily known by those skilled in the art from the content disclosed in the specification.

### Definition of Terms

In the present application, the term "oncolytic virus" generally refers to a virus that can replicate in tumor cells and kill the tumor cells. The oncolytic virus includes, but is not limited to, a vesicular stomatitis virus (referred to as "VSV"), a poxvirus, a herpes simplex virus (HSV), a measles virus, a Semliki Forest virus, a poliomyelitis virus, a reovirus, a Seneca Valley virus (SVV), an echovirus, a Coxsackie virus, a Newcastle disease virus (NDV), and a Maraba virus. In some embodiments, the oncolytic virus is modified to improve its selectivity for tumor cells. In some embodiments, the oncolytic virus is modified to reduce its immunogenicity.

In some embodiments, the VSV is a mutant of the VSV Indiana MuddSummer strain and can be used for tumor treatment. This virus does not interact with endogenous IFN-β in normal cells, and can only selectively amplify and grow only in tumor cells.

The VSV can express various cell surface molecules, including low density lipoprotein receptors, phosphatidylserine, sialolipid, and heparan sulfate, and can attach to the surface of cells through these molecules. Compared with other oncolytic viral platforms currently under development, the VSV possesses the following advantages: (1) a small genome, a short replication cycle, and rapid trans-synaptic spread; (2) extremely high expression of exogenous genes, enabling high titers and allowing for large-scale production; (3) an independent cell cycle, with no risk of transformation in the host cell's cytoplasm. This oncolytic virus does not integrate into the DNA, and after attenuation, it can avoid the neurological inflammation caused by the wild-type virus. Given these characteristics, the VSV holds great potential in tumor immunotherapy.

In some embodiments, site-directed gene mutations can be introduced into the M protein, and/or G protein, and/or N protein, and/or P protein, and/or L protein of the VSV.

In some embodiments, the recombinant oncolytic virus as described in the present application may be a genetically modified oncolytic virus, for example, being modified by altering one or more genes to enhance its selectivity for tumors and/or promote preferential replication in dividing cells. The genetic modifications may involve the alteration of genes involved in processes such as DNA/RNA replication, nucleic acid metabolism, host tropism, surface attachment, virulence, lysis, and dissemination. Alternatively, the modifications may involve the integration of exogenous genes. The exogenous genes may include exogenous immunomodulatory genes, exogenous screening genes, exogenous reporter genes, and the like. The modified oncolytic virus may also be those modified at the amino acid level, such as through the insertion, deletion, or substitution of one or more amino acids.

In the present application, the term "M protein" generally refers to the matrix protein of the VSV. M protein is an important virulence factor of VSV, also a known protein in the VSV that can interfere with the innate immune response of mice. The term "M protein" further includes a homolog, an ortholog, a variant, a functional active fragment, and the like thereof. In the present application, the M protein of the wild-type VSV Indiana MuddSummer strain may include an amino acid sequence as set forth in SEQ ID NO 1. In the present application, the M protein of the recombinant oncolytic virus may include an amino acid sequence as set forth in any one of SEQ ID NOs 2-11.

In the present application, the term "G protein" generally refers to the glycoprotein of VSV, also known as envelope protein. The term "G protein" further includes a homolog, an ortholog, a variant, a functional active fragment, and the like thereof. In the present application, the G protein of the wild-type VSV Indiana MuddSummer strain may include an amino acid sequence as set forth in SEQ ID NO 12. In the present application, the G protein of the recombinant oncolytic virus may include an amino acid sequence as set forth in SEQ ID NO 13.

In the present application, the term "N protein" generally refers to the nucleocapsid protein of VSV. The term "N protein" further includes a homolog, an ortholog, a variant, a functional active fragment, and the like thereof. In the present application, the N protein of the wild-type VSV Indiana MuddSummer strain may include an amino acid sequence as set forth in SEQ ID NO 14. In the present application, the N protein of the recombinant oncolytic virus may include an amino acid sequence as set forth in SEQ ID NO 15.

In the present application, the term "P protein" generally refers to the phosphoprotein of VSV. The term "P protein" further includes a homolog, an ortholog, a variant, a functional active fragment, and the like thereof. In the present application, the P protein of the wild-type VSV Indiana MuddSummer strain may include an amino acid sequence as set forth in SEQ ID NO 16. In the present application, the P protein of the recombinant oncolytic virus may include an amino acid sequence as set forth in SEQ ID NO 17.

In the present application, the term "L protein" generally refers to the RNA polymerase protein of VSV. The L gene of VSV encodes RNA poly E protein. The term "L protein" further includes a homolog, an ortholog, a variant, a functional active fragment, and the like thereof. In the present application, the L protein of the wild-type VSV Indiana MuddSummer strain may include an amino acid sequence as set forth in SEQ ID NO 18. In the present application, the L protein of the recombinant oncolytic virus may include an amino acid sequence as set forth in SEQ ID NO 19.

In the present application, a mutation site of a protein is generally expressed as "amino acid + position of the amino acid + mutated amino acid". In the present application, the mutation may include, but is not limited to, the addition, substitution, and/or deletion of amino acids. For example, the term "M51R" generally refers to a mutation of methionine at position 51 to arginine R.

In the present application, the term "amino acid substitution" generally refers to replacing an amino acid residue present in the parental sequence with another amino acid residue. The amino acid in the parental sequence can be substituted, for example, via chemical synthesis or by recombination methods known in the art. Therefore, "substitution at position xx" generally refers to replacing the amino acid present at position xx with a substitute amino acid residue. In the present application, the amino acid substitution may include amino acid mutations.

In the present application, the term "mutation" generally refers to an alteration in the nucleotide or amino acid sequence of a wild-type molecule. Amino acid changes may include substitution, deletion, insertion, addition, truncation of an amino acid, or processing and cleavage of the protein.

In the present application, the recombinant oncolytic virus is obtained by integrating an exogenous gene while introducing site-directed gene mutations into the M protein, and/or G protein, and/or N protein, and/or P protein, and/or L protein of the VSV. The exogenous gene is specifically a gene encoding an antigen and/or a cytokine.

Macromolecular drugs, also known as biologics, refer to pharmaceuticals used for the prevention, treatment, and diagnosis of human diseases, which are prepared from biological materials such as microorganisms, cells, and various animal- or human-derived tissues and fluids, obtained through conventional means or biotechnologies including genetic engineering, cell engineering, protein engineering, and fermentation engineering.

The mechanism of action of macromolecular drugs differs from that of conventional or small-molecule drugs. Macromolecular drugs primarily exert their effects by stimulating the body's immune system to produce immune substances (such as antibodies), thereby inducing humoral immunity, cellular immunity, or cell-mediated immunity within the human body. Successfully launched macromolecular drugs include Trastuzumab, Zenapax, Rituximab, etc. Common biological macromolecular drugs include polypeptides, proteins, antibodies, glycans, and nucleic acids. Clinically, they are often developed into antibodies and vaccines. In the present application, the "macromolecular antibody-based anti-cancer drug" is an antibody-based anti-cancer drug.

In some specific embodiments, the macromolecular antibody-based anti-cancer drug may include, but is not limited to:
1. A macromolecular antibody-based anti-cancer drug targeting HER2:
   Trastuzumab, Trade name: Herceptin (an antibody-based drug); drug type: humanized; target: HER2; FDA-approved indications: HER2-positive metastatic/non-metastatic breast cancer; HER2-positive metastatic gastric or gastroesophageal junction adenocarcinoma.
   Pertuzumab, Trade name: Perjeta (an antibody-based drug); drug type: humanized; target: HER2; FDA-approved indications: HER2-positive metastatic breast cancer; HER2-positive, locally advanced, inflammatory or early breast cancer.
   Ado-trastuzumab emtansine, Trade name: Kadcyla; drug type: humanized; target: HER2; FDA-approved indications: HER2-positive metastatic breast cancer and lung cancer.
   Disitamab, target: HER2; FDA-approved indications: gastric cancer and esophageal cancer.
   Margetuximab, target: HER2; FDA-approved indication: breast cancer.
   Inetetamab, target: HER2; FDA-approved indication: breast cancer.
   Fam-trastuzumab deruxtecan-nxki (Trastuzumab recombinant freeze-dried powder), target: HER2; FDA-approved indications: lung cancer, gastric cancer, and breast cancer.
   Trastuzumab deruxtecan, target: HER2; FDA-approved indication: breast cancer.
   Disitamab vedotin, target: HER2; FDA-approved indications: urothelial carcinoma, gastric cancer, and gastroesophageal junction cancer.
2. A macromolecular antibody-based anti-cancer drug targeting EGFR:
   Cetuximab, Trade name: Erbitux; drug type: chimeric; target: EGFR; FDA-approved indications: metastatic colorectal cancer, head and neck squamous cell carcinoma, gastric cancer, and esophageal cancer.
   Panitumumab, Trade name: Vectibix; drug type: human; target: EGFR; FDA-approved indication: metastatic colorectal cancer.
   Necitumumab, Trade name: Portrazza; drug type: human; target: EGFR; FDA-approved indication: metastatic squamous NSCLC (non-small cell lung cancer).
   Nimotuzumab, target: EGFR; FDA-approved indications: head and neck cancer and pancreatic cancer.
   Amivantamab, targets: EGFR and Met; FDA-approved indication: lung cancer.
   Getuximab Saratolacan, target: EGFR; FDA-approved indication: head and neck cancer.
3. A macromolecular antibody-based anti-cancer drug targeting PD-1:
   Nivolumab, Trade name: Opdivo; drug type: human; target: PD-1; FDA-approved indications: unresectable or metastatic melanoma, metastatic squamous NSCLC, metastatic NSCLC, advanced renal cell carcinoma, recurrent or metastatic head and neck squamous cell carcinoma, lymphoma, mesothelioma, bladder cancer, liver cancer, head and neck cancer, gastric cancer, esophageal cancer, biliary tumor, Merkel cell carcinoma, and thymoma.
   Pembrolizumab, Trade name: Keytruda; drug type: humanized; target: PD-1; FDA-approved indications: unresectable or metastatic melanoma, metastatic NSCLC, recurrent metastatic head and neck squamous cell carcinoma, lymphoma, solid tumor, lung cancer, Merkel cell carcinoma, cervical cancer, endometrial carcinoma, kidney cancer, bladder cancer, liver cancer, head and neck cancer, gastric cancer, esophageal cancer, biliary tumor, prostate cancer, alveolar soft part sarcoma, undifferentiated pleomorphic sarcoma, and thymoma.
   Tislelizumab, target: PD-1; FDA-approved indications: lung cancer, liver cancer, bladder cancer, and lymphoma.
   Sintilimab, target: PD-1; FDA-approved indications: lung cancer, liver cancer, and lymphoma.
   Cemiplimab, target: PD-1; FDA-approved indications: lung cancer and melanoma.
   Toripalimab, target: PD-1; FDA-approved indications: nasopharyngeal carcinoma, bladder cancer, melanoma, and head and neck cancer.
   Camrelizumab, target: PD-1; FDA-approved indications: nasopharyngeal carcinoma, esophageal cancer, liver cancer, biliary tumor, kidney cancer, head and neck cancer, and lymphoma.
   Dostarlimab, target: PD-1; FDA-approved indication: endometrial carcinoma.
   Penpulimab, target: PD-1; FDA-approved indication: lymphoma.
   Zimberelimab, target: PD-1; FDA-approved indication: lymphoma.
   Cadonilimab, targets: PD-1 and CTLA4; FDA-approved indication: cervical cancer.
4. A macromolecular antibody-based anti-cancer drug targeting PD-L1:
   Atezolizumab, Trade name: Tecentriq; drug type: humanized; target: PD-L1; FDA-approved indications: locally advanced or metastatic urothelial carcinoma, metastatic NSCLC, melanoma, breast cancer, bladder cancer, and liver cancer.
   Durvalumab, target: PD-L1; FDA-approved indications: lung cancer and bladder cancer.
   Avelumab, target: PD-L1; FDA-approved indications: Merkel cell carcinoma, kidney cancer, and bladder cancer.
   Sugemalimab, target: PD-L1; FDA-approved indication: lung cancer.
5. A macromolecular antibody-based anti-cancer drug targeting TROP2:
   Sacituzumab govitecan, Trade name: Trodelvy; target: TROP2; FDA-approved indications: breast cancer and bladder cancer.
6. A macromolecular antibody-based anti-cancer drug targeting BCMA:
   Belantamab Mafodotin, target: BCMA; FDA-approved indication: multiple myeloma.
   Elranatamab, targets: BCMA and CD3; FDA-approved indication: multiple myeloma.
   Teclistamab; targets: BCMA and CD3; FDA-approved indication: multiple myeloma.
7. A macromolecular antibody-based anti-cancer drug targeting VEGFR-2:
   Ramucirumab, Trade name: Ciramza; drug type: human; target: VEGFR-2; FDA-approved indications: advanced or metastatic gastroesophageal junction adenocarcinoma, metastatic NSCLC, metastatic colorectal cancer, lung cancer, liver cancer, gastric cancer, and esophageal cancer.
8. A macromolecular antibody-based anti-cancer drug targeting SLAMF7:
   Elotuzumab, target: SLAMF7 (CS1/CD319/CRACC); FDA-approved indication: multiple myeloma.
9. A macromolecular antibody-based anti-cancer drug targeting CD3:
   Blinatumomab, targets: CD3 and CD19; FDA-approved indication: leukemia.
   Elranatamab, targets: BCMA and CD3; FDA-approved indication: multiple myeloma.
   Teclistamab, targets: BCMA and CD3; FDA-approved indication: multiple myeloma.
   Talquetamab, targets: CD3 and GPRC5D; FDA-approved indication: multiple myeloma.
   Epcoritamab, targets: CD20 and CD3; FDA-approved indications: diffuse large B cell lymphoma, high grade B cell lymphoma, mediastinal large B cell lymphoma, and follicular lymphoma.
   Glofitamab, targets: CD20 and CD3; FDA-approved indication: diffuse large B cell lymphoma.
   Mosunetuzumab, targets: CD20, CD3; FDA-approved indication: follicular lymphoma.
   Catumaxomab, targets: EPCAM and CD3; FDA-approved indication: malignant effusions.
10. A macromolecular antibody-based anti-cancer drug targeting CD19:
   Blinatumomab, targets: CD3 and CD19; FDA-approved indication: leukemia.
   Loncastuximab tesirine, target: CD19; FDA-approved indication: B-cell lymphoma.
11. A macromolecular antibody-based anti-cancer drug targeting CD20:
   Rituximab, target: CD20; FDA-approved indication: lymphoma.
   Obinutuzumab, target: CD20; FDA-approved indications: lymphoma and leukemia.
   Tosituomab, target: CD20; FDA-approved indication: lymphoma.
   Ofatumumab, target: CD20; FDA-approved indication: leukemia.
   Ibritumomab, target: CD20; FDA-approved indication: lymphoma.
   Ibritumomab tiuxetan, target: CD20; FDA-approved indications: multiple hematological diseases.
   Epcoritamab, targets: CD20 and CD3; FDA-approved indications: diffuse large B cell lymphoma, high grade B cell lymphoma, mediastinal large B cell lymphoma, and follicular lymphoma.
   Glofitamab, targets: CD20 and CD3; FDA-approved indication: diffuse large B cell lymphoma.
   Mosunetuzumab, targets: CD20 and CD3; FDA-approved indication: follicular lymphoma.
12. A macromolecular antibody-based anti-cancer drug targeting CD22:
   Inotuzumab Ozogamicin, target: CD22; FDA-approved indication: leukemia.
   Moxetumomab Pasudotox, target: CD22; FDA-approved indication: leukemia.
13. A macromolecular antibody-based anti-cancer drug targeting CD30:
   Brentuximab (Brentuximab vedotin), target: CD30; FDA-approved indication: lymphoma.
14. A macromolecular antibody-based anti-cancer drug targeting CD33:
   Gemtuzumab, target: CD33; FDA-approved indication: leukemia.
   Gemtuzumab ozogamicin, target: CD33; FDA-approved indication: acute myeloid leukemia.
15. A macromolecular antibody-based anti-cancer drug targeting CD38:
   Daratumumab, target: CD38; FDA-approved indication: multiple myeloma.
16. A macromolecular antibody-based anti-cancer drug targeting CD52:
   Alemtuzumab, target: CD52; FDA-approved indication: leukemia.
17. A macromolecular antibody-based anti-cancer drug targeting CD79b:
   Polatuzumab vedotin, target: CD79b; FDA-approved indications: lymphoma and leukemia.
18. A macromolecular antibody-based anti-cancer drug targeting Met:
   Amivantamab, targets: EGFR and Met; FDA-approved indication: lung cancer.
19. A macromolecular antibody-based anti-cancer drug targeting GD2:
   Dinutuximab, Trade name: Unituxin; drug type: Chimeric; target: GD2; FDA-approved indications: pediatric high-risk neuroblastoma and central nervous system sarcoma.
20. A macromolecular antibody-based anti-cancer drug targeting VEGF:
   Bevacizumab, Trade name: Avastin; drug type: humanized; target: VEGF; FDA-approved indications: metastatic colorectal cancer, recurrent or metastatic non-squamous NSCLC, metastatic renal cell carcinoma, persistent, recurrent or metastatic cervical glioblastoma, recurrent cancer, epithelial ovarian cancer, fallopian tube carcinoma or primary peritoneal cancer, lung cancer, liver cancer, angiosarcoma, hemangiopericytoma, and central nervous system sarcoma.
   Byvasda, target: VEGF; FDA-approved indication: central nervous system sarcoma.
21. A macromolecular antibody-based anti-cancer drug targeting PDGFR-α:
   Olaratumab, Trade name: Lartruvo; drug type: human; target: PDGFR-α; FDA-approved indication: soft tissue sarcoma.
22. A macromolecular antibody-based anti-cancer drug targeting CTLA-4:
   Ipilimumab, Trade name: Yervoy; drug type: human; target: CTLA-4; FDA-approved indications: unresectable or metastatic melanoma, cutaneous melanoma, colorectal cancer, mesothelioma, kidney cancer, liver cancer, and thymoma.
   Cadonilimab, targets: PD-1 and CTLA4; FDA-approved indication: cervical cancer.
23. A macromolecular antibody-based anti-cancer drug targeting RANKL:
   Denosumab, Trade name: Xgeva; drug type: human; target: RANKL; FDA-approved indications: osseous metastasis of solid tumor, and giant-cell tumor of bone.
24. A macromolecular antibody-based anti-cancer drug targeting FRa:
   Mirvetuximab: target: FRa; FDA-approved indication: ovarian cancer.
   Mirvetuximab Soravtansine, target: FRα; FDA-approved indications: ovarian cancer, fallopian tube carcinoma, and peritoneal cancer.
25. A macromolecular antibody-based anti-cancer drug targeting TF:
   Tisotumab vedotin, target: TF; FDA-approved indication: cervical cancer.
26. A macromolecular antibody-based anti-cancer drug targeting IL6:
   Siltuximab, target: IL6; FDA-approved indication: lymphoma.
27. A macromolecular antibody-based anti-cancer drug targeting GPRC5D:
   Talquetamab, targets: CD3 and GPRC5D; FDA-approved indication: multiple myeloma.
28. A macromolecular antibody-based anti-cancer drug targeting TNF-α:
   Ozoralizumab, targets: TNF-α and Albumin; FDA-approved indication: rheumatoid arthritis.
29. A macromolecular antibody-based anti-cancer drug targeting EPCAM:
   Catumaxomab, target: EPCAM and CD3; FDA-approved indication: malignant effusions.
30. A macromolecular antibody-based anti-cancer drug targeting 5T4:
   Anti-5T4 antibody [EPR5529] (ab134162), an antibody purchased from abcam, targeting 5T4 (TPBG).
   GEN-1044 (Genmab), indications: bladder cancer, esophageal cancer, non-small cell lung cancer, prostate cancer, triple-negative breast cancer, and uterine cancer; drug type: bispecific antibody.
   ALG.APV-527 (Aptevo Therapeutics, Inc. Alligator Bioscience AB), indications: solid tumors; drug type: bispecific antibody.
   SYD-1875 (Synthon), indications: solid tumors; drug type: ADC.
   ASN-004 (Asana BioSciences LLC), indications: solid tumors, colorectal cancer, metastatic breast cancer, non-small cell lung cancer, and ovarian cancer; drug type: ADC.
   CBA-1535, indications: tumors; drug type: trispecific antibody.
   DM004 (Xadcera), Indication: solid tumors; drug type: bispecific antibody ADC.
31. A macromolecular antibody-based anti-cancer drug targeting B7-H3:
   Anti-CD276 antibody [EPNCIR122] (ab134161), purchased from abcam, targeting B7-H3(CD276).
   Omburtamab, indications: central nervous system tumor metastases (Phase II/III), meningeal tumors (Phase II/III), neuroblastoma (Phase II/III), and non-progressive diffuse intrinsic pontine glioma (Phase I).
   Enoblituzumab, indications: head and neck squamous cell carcinoma (Phase II) and melanoma (Phase I).
   Omburtamab, indications: tumors (Phase I/II) and pediatric neuroblastoma (Phase I/II).
   MGC018, indications: tumors (Phase I/II).
   Obrindatamab, indications: tumors (Phase I).
   Mirzotamab clezutoclax, indications: tumors (Phase I).
   TAK 280, indications: tumors (Phase I).
   YBL 018, indications: tumors (Phase I).
   ATG 027, indications: tumors (Preclinical) and hematologic tumors (Preclinical).
   MIL108, indications: tumors (Preclinical).
   ITC 6146RO, indications: tumors (Preclinical).
   GTB 5550, indications: tumors (Preclinical).
   Hu8H9, indications: tumors (Preclinical).
   SHR-1812, indications: tumors (Preclinical).
   XmAb 808, indications: tumors (Preclinical).
   BAT 8009, indications: tumors (Preclinical).
   HS-20093, indications: tumors (Phase I).
32. A macromolecular antibody-based anti-cancer drug targeting GPC3.
   Anti-Glypican 3 antibody [SP86] (ab95363), an antibody purchased from abcam, targeting GPC3.
   ECT-204, indications: liver cancer and hepatocellular carcinoma.
   Codrituzumab/GC33 (Chugai Pharmaceutical Co., Ltd.; Roche), Indication: hepatocellular carcinoma.
   GPC-3298306 (National. Cancer Center. Of Japan), indications: hepatocellular carcinoma and ovarian cancer.
   ERY-974 (Chugai Pharmaceutical Co., Ltd.), indications: solid tumors.
   MDX-1414 (Bristol Myers Squibb), indications: tumors.
   B010-A (Shanghai Pharmaceuticals Holding Co., Ltd.), Indication: hepatocellular carcinoma.
   HLX-63 (Shanhai Henlius Biotech, Inc.), indications: solid tumors.
   LQ-102, Indication: hepatocellular carcinoma.
33. A macromolecular antibody-based anti-cancer drug targeting Claudin 18.2:
   Anti-Claudin18.2 antibody [EPR19202] (ab222512), an antibody purchased from abcam, targeting Claudin 18.2.
   Zolbetuximab (Astellas Pharma China, Inc.), indications: esophageal cancer, gastric cancer, adenocarcinoma, pancreatic cancer, gastrointestinal diseases, cystic lymphangitic carcinomatosis, pain, and solid tumors; Drug type: Chimeric monoclonal antibody.
   Q-1802 (QureBio Ltd.), indications: solid tumors; drug type: Bispecific antibody.
   LM-102 (LaNova Medicines), indications: solid tumors; drug type: Biologics.
   TJ-CD48 (I-Mab Biopharma Co., Ltd.), indications: tumors; drug type: Bispecific antibody.
   Recombinant humanized anti-Claudin 18.2 monoclonal antibody (CARsgen Therapeutics Ltd.), indications: solid tumors, gastric cancer, and pancreatic cancer; drug type: humanized monoclonal antibody.
   Claudin18.2 humanized monoclonal antibody (Mabspacebio) (Mabspace Biosciences Co., Ltd), indications: gastric cancer, solid tumors, tumors, bile duct cancer, gallbladder carcinoma, lung cancer, and esophageal cancer; drug type: humanized monoclonal antibody.
   HBM-1029 (Harbour BioMed), indications: solid tumors; drug type: Monoclonal antibody.
   HLX-58 (Shanhai Henlius Biotech, Inc.), indications: solid tumors.
   SOT-102 (Sotio), indications: solid tumors; drug type: antibody-drug conjugate.

In some specific embodiments, the polyclonal antibody-based anti-cancer drug may include, but is not limited to:
Amivantamab, Blinatumomab, Ofatumumab, Elranatamab, Teclistamab, Talquetamab, Epcoritamab, Glofitamab, Cadonilimab, Mosunetuzumab, Ozoralizumab, and Catumaxomab.
In addition, the polyclonal antibody-based anti-cancer drug further includes the following:
Enfortumab vedotin, target: Nectin-4; FDA-approved indication: urothelial carcinoma.
Emicizumab, target: FIX; FDA-approved indications: hemophilia A, acquired hemophilia A, and hemorrhage.

In some specific embodiments, the antibody-drug conjugate may include, but is not limited to:
Ado-trastuzumab emtansine, Fam-trastuzumab deruxtecan-nxki, Trastuzumab deruxtecan, etuximab Saratolacan, Tisotumab vedotin, Loncastuximab tesirine, Belantamab mafodotin, Sacituzumab govitecan, Moxetumomab pasudotox, Inotuzumab ozogamicin, Brentuximab Vedotin, Mirvetuximab Soravtansine, Disitamab vedotin, Polatuzumab vedotin, and Gemtuzumab ozogamicin.

In the present application, the term "monoclonal antibody" refers to highly homogeneous antibodies produced by a single B-cell clone, which recognize only a specific epitope of an antigen.

In the present application, the term "polyclonal antibody" refers to antibodies produced by multiple B-lymphocyte clones, stimulated by multiple epitopes, and capable of binding to various antigenic epitopes. To some extent, polyclonal antibodies are a mixture of multiple monoclonal antibodies.

In the present application, the term "antibody-drug conjugate" (ADC) refers to the conjugation of a highly target-specific antibody and a highly potent chemotherapeutic drug, enabling the precise delivery of the drug into tumor cells while avoiding damage of the chemotherapeutic drug to normal cells, thereby reducing adverse effects during the treatment. The first ADC drug, Mylotarg, was approved for marketing in 2000. Currently approved ADC drugs include -trastuzumab emtansine, Trastuzumab deruxtecan, Sacituzumab govitecan, Ibritumomab tiuxetan, Inotuzumab Ozogamicin, Moxetumomab Pasudotox, Brentuximab, Gemtuzumab ozogamicin, Polatuzumab vedotin, and Enfortumab vedotin.

In the present application, the term "antigen" refers to a substance capable of inducing the production of antibodies or immune cells, that is, any substance that can elicit an immune response in the body. Specifically, the antigen is a substance that can be specifically recognized and bound by antigen receptors (TCR/BCR) on the surface of T/B lymphocytes, thereby activating T/B cells to proliferate and differentiate, producing immune response products (sensitized lymphocytes or antibodies), with which the antigen can bind specifically both in vivo and in vitro. Therefore, antigenic substances possess two important properties: immunogenicity and immunoreactivity. Immunogenicity refers to the ability of an antigen to induce a specific immune response in the body, resulting in the production of antibodies and/or sensitized lymphocytes. Immunoreactivity refers to the ability to specifically bind to the corresponding immune effector molecules (antibodies or sensitized lymphocytes) both in vivo and in vitro.

In some specific embodiments, the antigen is exogenous, meaning that the antigen is derived from a different species.

In some specific embodiments, the antigen is an endogenous antigen. Specifically, the antigen is generally an antigen expressed on the tumor cells.

In a specific embodiment, the antigen is a tumor-associated antigen (TAA) or a tumor specific antigen (TSA).

In a specific embodiment, TAA or TSA encompasses molecules, or parts thereof, that are presented on the cell surface (antigens recognized by CARs), within the cell membrane (antigens recognized by TCRs), or present in the tumor environment (e.g., in the tumor microenvironment).

In some specific embodiments, the cells are tumor cells.

In some specific embodiments, TAA or TSA includes tumor-associated antigens or tumor specific antigens on the cell surface or within the cell membrane.

In some specific embodiments, the cells are non-tumor cells present in the tumor environment, for example, but not limited to cells within the vascular system tissues associated with tumors or cancers.

In some specific embodiments, TAA or TSA are antigens associated with angiogenesis in the tumor microenvironment.

In some specific embodiments, TAA or TSA are antigens on the vasculature in the tumor microenvironment.

In some specific embodiments, the cells are stromal cells present in the tumor environment.

In some specific embodiments, TAA or TSA are stromal cell antigens in the tumor microenvironment.

In some specific embodiments, TAA or TSA includes extracellular epitopes of antigens on the tumor cell surface, tetramers within or outside the tumor cell membrane, or other structures that can be recognized by antibodies or immune cells.

In some specific embodiments, TAA or TSA includes extracellular stromal antigens.

In some specific embodiments, TAA or TSA includes antigens present in the tumor microenvironment (TME).

In some specific embodiments, TAA or TSA includes molecules secreted by tumor cells into the TME.

In some specific embodiments, TAA or TSA includes effector molecules secreted by tumor cells into the TME.

In some specific embodiments, TAA or TSA includes effector molecules secreted by tumor cells into the TME to downregulate or inhibit the activity of cytotoxic natural killer (NK) cells or T cells.

In some specific embodiments, TAA or TSA includes soluble activating receptor ligands secreted by tumor cells into the TME to block the recognition of tumor cells by NK cells or T cells.

In some specific embodiments, the examples of TAA or TSA include, but are not limited to, 5T4, RORl, EGFR, FcγRI, FcγRIIa, FcγRIIb, FcγRIIIa, FcγRIIIb, CD24, CD28, CD137, CTLA-4, FAS, FAP (fibroblast activation protein), LGR5, C5aR1, A2AR, fibroblast growth factor receptor 1 (FGFR1), FGFR2, FGFR3, FGFR4, glucocorticoid-induced TNFR-related (GITR) protein, lymphotoxin-β receptor (LTβR), a toll-like receptor (TLR), tumor necrosis factor-related apoptosis-inducing ligand receptor 1 (TRAIL receptor 1), TRAIL receptor 2, prostate specific membrane antigen (PSMA) protein, prostate stem cell antigen (PSCA) protein, tumor-associated protein carbonic anhydrase IX (CAIX), epidermal growth factor receptor 1 (EGFR1), EGFRvIII, human epidermal growth factor receptor 2 (HER2/neu; Erb2), ErbB3 (Her3), a folate receptor, an ephrin receptor, PDGFRa, ErbB2, CD2, CD20, CD22, CD30, CD33, CD40, CD37, CD38, CD70, CD74, CD56, CD80, CD86, CD123, CCAM5, CCAM6, BCMA, p53, MET (tyrosine protein kinase, Met), hepatocyte growth factor receptor (HGFR), MAGE-A1, MAGE-A2, MAGE-A3, MAGE-A4, MAGE-A6, MAGE-A10, MAGE-A12, BAGE, DAM-6, DAM-10, GAGE-1, GAGE-2, GAGE-8, GAGE-3, GAGE-4, GAGE-5, GAGE-6, GAGE-7B, NA88-A, NY-ESO-1, BRCA1, BRCA2, MART-1, MC1R, Gp100, PSA, PSM, tyrosinase, Wilms tumor antigen (WT1), TRP-1, TRP-2, ART-4, CAMEL, Cyp-B, hTERT, hTRT, iCE, MUC1, MUC2, P-cadherin, myostatin (GDF8), Cripto (TDGF1), MUC5AC, PRAME, P15, RU1, RU2, SART-1, SART-3, WT1, AFP, β-catenin/m, caspase-8/m, CDK-4/m, ELF2M, GnT-V, G250, HSP70-2M, HST-2, KIAA0205, MUM-1, MUM-2, MUM-3, myosin/m, RAGE, SART-2, TRP-2/INT2, 707-AP, annexin II, CDC27/m, TPI/mbcr-abl, ETV6/AML, LDLR/FUT, Pml/RARα, TEL/AML1, CD28, CD137, CanAg, mesothelin, DR5, PD-1, PD-L1, HER2, HER3, IGF-1R, CXCR4, neuropilin 1, glypicans, EphA2, CD138, B7-H3, B7-H4, gpA33, GPC3, SSTR2 or VEGF-R2, CEA, EpCAM, Nectin-4, KARS, CD39, CD73, TIGIT, CD47, DLL3, and Claudin 18.1.

In the present application, the term "cytokine" refers to biologically active substances that are synthesized and secreted by immune cells (lymphocytes, mononuclear macrophages, etc.) and related cells (vascular endothelial cells, fibroblasts, etc.) to regulate the functions of other immune cells or target cells, which are macromolecular polypeptides or glycoproteins. Cytokines with immunomodulatory effects can all be expressed via recombinant oncolytic viruses. Based on their primary functions, cytokines are classified into interleukin (IL), interferon (IFN), tumor necrosis factor (TNF), colony stimulating factor (CSF), transforming growth factor-β family (TGF-β family), growth factor (GF), and chemokine family.

Interleukin includes IL-1, IL-2, IL-7, IL-9, IL-15, IL-21, IL-4, IL-12, and IL-18.

Specifically, interleukin 12 (IL-12): IL-12 acts on activated T and NK cells and exhibits a broad spectrum of biological activities, exerting its effects on lymphocytes through mediation by the activator of the transcription protein STAT4. IL-12 is essential for the T cell-independent induction of IFN-γ and plays an important role in the differentiation of Th1 and Th2 cells. IL12B binds to IL23A to form the IL-23 interleukin, which possesses both innate and adaptive immune function. IL-12 is considered a drug target. In cellular immunotherapy, IL-12 promotes CD4 + T cells to differentiate into CD4 + Th1 T cells and enhances the activity of CD8 + CTLs cells. The therapeutic effects of IL-12 depend on its doses, duration of action, and interactions with other cytokines, promoting the tumoricidal activity of immune cells through multiple mechanisms. In murine anti-melanoma models, high-dose IL-12 exerts its effects via NK cells, while low-dose IL-12 mediates tumor killing via NKT cells.

Specifically, interleukin 18 (IL-18): IL-18, also known as interferon-γ inducing factor, is a proinflammatory cytokine produced by macrophages and other cells. IL-18 can stimulate NK cells and CD8 + T cells to secrete IFN-γ and enhance the cytotoxic effects of NK cells and CD8 + T cells. IL-18 can also activate macrophages, promote the development of Th1 CD4 + T cells, and facilitate the expression of FasL by lymphocytes. IL-18 can provide potential therapeutic targets for allergic diseases. In addition, IL-18 acts synergistically with IL-12 and IL-15 to sustain Th1 responses and monokine production in autoimmune diseases.

In the present application, the term "nucleic acid molecule" generally refers to a nucleotide of any length. In the present application, the term "nucleic acid molecule" can encode proteins contained in the oncolytic virus. In the present application, the nucleic acid molecule may include DNAand/or RNA. In some cases, the RNA may include a single-stranded RNA (ssRNA) or a double-stranded RNA (dsRNA), and the single-stranded RNA may include sense RNA, anti-sense RNA, or ambisense RNA.

In the present application, the term "prevention" generally refers to preventing the occurrence, onset, recurrence, and/or spread of a disease or one or more symptoms thereof by taking certain measures in advance. In the present application, the term "treatment" generally refers to eliminating or ameliorating a disease or one or more symptoms associated with the disease. In some embodiments, "treatment" generally refers to administering one or more drugs to a patient suffering from such a disease to eliminate or alleviate the disease. In some embodiments, "treatment" may involve administering the pharmaceutical combination and/or pharmaceutical product after the onset of symptoms of a specific disease, in the presence or absence of other drugs. For example, the pharmaceutical combination and/or pharmaceutical product of the present application is used to prevent the occurrence, development, recurrence, and/or metastasis of a tumor.

In the present application, the term "tumor" generally refers to any new pathological tissue hyperblastosis. The tumor may be benign or malignant. In the present application, the tumor may be a solid tumor and/or a hematological tumor. For research purposes, these tissues can be isolated from readily available resources using methods well known to those skilled in the art.

In some specific embodiments, the tumor includes, but is not limited to, acute lymphoblastic leukemia, acute B lymphocytic leukemia, chronic non-lymphocytic leukemia, non-Hodgkin lymphoma, anal cancer, astrocytoma, basal cell carcinoma, bile duct cancer, bladder cancer, mastocarcinoma, breast cancer (BRCA), cervical cancer, chronic myeloproliferative neoplasm, colorectal cancer, endometrial carcinoma, ependymoma, esophageal cancer, diffuse large B cell lymphoma (DLBCL), esthesioneuroblastoma, Ewing's sarcoma, fallopian tube carcinoma, gallbladder carcinoma, gastric cancer, gastrointestinal carcinoid tumor, hepatocellular carcinoma, hypopharyngeal carcinoma, Kaposi's sarcoma, kidney cancer, Langerhans cell histiocytosis, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumor, pharyngeal carcinoma, pituitary adenoma, prostate cancer, rectal cancer, renal cell carcinoma, retinoblastoma, skin cancer, small cell lung cancer, small intestine cancer, neck squamous cell carcinoma, testicular cancer, thymoma, thyroid cancer, uterine cancer, vaginal cancer, and hemangioma.

### Detailed Description of the Invention

A wild-type VSV is specifically the VSV Indiana serotype, the VSV Indiana MuddSummer strain. The amino acid sequence of the M protein of the wild-type VSV is as set forth in SEQ ID NO 1; the amino acid sequence of the G protein of the wild-type VSV is as set forth in SEQ ID NO 12; the amino acid sequence of the N protein of the wild-type VSV is as set forth in SEQ ID NO 14; the amino acid sequence of the P protein of the wild-type VSV is as set forth in SEQ ID NO 16; and the amino acid sequence of the L protein of the wild-type VSV is as set forth in SEQ ID NO 18. In the present application, the M protein, G protein, N protein, P protein, and L protein of the wild-type VSV all can be modified.

A recombinant oncolytic virus, which is derived from the above wild-type VSV and is obtained through site-directed mutations in the amino acid sequences of the M, G, N, P, and L proteins.

The present application provides a method for treating a tumor with a combination of a recombinant oncolytic virus and a macromolecular antibody-based anti-cancer drug, which specifically includes using the macromolecular antibody-based anti-cancer drug and the recombinant oncolytic virus in combination to treat the tumor.

The macromolecular antibody-based anti-cancer drug includes, but is not limited to, a macromolecular antibody-based anti-cancer drug targeting HER2, a macromolecular antibody-based anti-cancer drug targeting EGFR, a macromolecular antibody-based anti-cancer drug targeting PD-1, a macromolecular antibody-based anti-cancer drug targeting PD-L1, a macromolecular antibody-based anti-cancer drug targeting TROP2, a macromolecular antibody-based anti-cancer drug targeting BCMA, a macromolecular antibody-based anti-cancer drug targeting VEGFR-2, a macromolecular antibody-based anti-cancer drug targeting SLAMF7, a macromolecular antibody-based anti-cancer drug targeting CD3, a macromolecular antibody-based anti-cancer drug targeting CD19, a macromolecular antibody-based anti-cancer drug targeting CD20, a macromolecular antibody-based anti-cancer drug targeting CD22, a macromolecular antibody-based anti-cancer drug targeting CD30, a macromolecular antibody-based anti-cancer drug targeting CD33, a macromolecular antibody-based anti-cancer drug targeting CD38, a macromolecular antibody-based anti-cancer drug targeting CD52, a macromolecular antibody-based anti-cancer drug targeting CD79b, a macromolecular antibody-based anti-cancer drug targeting Met, a macromolecular antibody-based anti-cancer drug targeting GD2, a macromolecular antibody-based anti-cancer drug targeting VEGF, a macromolecular antibody-based anti-cancer drug targeting PDGFR-α, a macromolecular antibody-based anti-cancer drug targeting CTLA-4, a macromolecular antibody-based anti-cancer drug targeting RANKL, a macromolecular antibody-based anti-cancer drug targeting FRa, a macromolecular antibody-based anti-cancer drug targeting TF, a macromolecular antibody-based anti-cancer drug targeting IL6, a macromolecular antibody-based anti-cancer drug targeting GPRC5D, a macromolecular antibody-based anti-cancer drug targeting TNF-α, a macromolecular antibody-based anti-cancer drug targeting EPCAM, a macromolecular antibody-based anti-cancer drug targeting CD24, a macromolecular antibody-based anti-cancer drug targeting 5T4, a macromolecular antibody-based anti-cancer drug targeting B7-H3, a macromolecular antibody-based anti-cancer drug targeting GPC3, and a macromolecular antibody-based anti-cancer drug targeting Claudin 18.2.

The recombinant oncolytic virus includes an M protein, a G protein, an N protein, a P protein, and an L protein.

Compared with an amino acid sequence as set forth in SEQ ID NO 1, an site mutation of the M protein includes one or more of M51R, V221F, or S226R; alternatively, the site mutation of the M protein includes one or more of N32S, N49D, M51R, H54Y, V221F, V225I, or S226R; alternatively, the site mutation of the M protein includes one or more of N32S, N49D, M51R, H54Y, knocking out of nucleotide bases encoding leucine at position 111, V221F, V225I, or S226R; alternatively, the site mutation of the M protein includes one or more of N32S, N49D, M51R, H54Y, L111A, V221F, V225I, or S226R; alternatively, the site mutation of the M protein includes one or more of G21E, N32S, N49D, M51R, H54Y, V221F, V225I, or S226R; alternatively, the site mutation of the M protein includes one or more of G21E, N32S, M33A, N49D, M51R, H54Y, V221F, V225I, or S226R; alternatively, the site mutation of the M protein includes one or more of G21E, N32S, M33A, N49D, M51R, H54Y, A133T, V221F, V225I, or S226R; alternatively, the site mutation of the M protein includes one or more of N32S, M33A, N49D, M51R, H54Y, V221F, V225I, or S226R; alternatively, the site mutation of the M protein includes one or more of N32S, M33A, N49D, M51R, H54Y, A133T, V221F, V225I, or S226R; alternatively, the site mutation of the M protein includes one or more of N32S, N49D, M51R, H54Y, A133T, V221F, V225I, or S226R.

Compared with an amino acid sequence as set forth in SEQ ID NO 12, an site mutation of the G protein includes one or more of V53I, A141V, D172Y, K217E, D232G, V331A, V371E, G436D, T438S, F453L, T471I, or Y487H.

Compared withan amino acid sequence as set forth in SEQ ID NO 14, an site mutation of the N protein includes one or more of I14V, R155K, or S353N.

Compared with an amino acid sequence as set forth in SEQ ID NO 16, an site mutation of the P protein includes one or more of R50K, V76A, D99E, L126S, L140S, H151Y, I168M, K170E, Y189S, or N237D.

Compared with an amino acid sequence as set forth in SEQ ID NO 18, an site mutation of the L protein includes one or more of S87P or I487T.

Furthermore, the recombinant oncolytic virus is obtained by introducing an exogenous gene encoding an antigen based on the above recombinant oncolytic virus.

Furthermore, the macromolecular antibody-based anti-cancer drug is one or more selected from a group consisting of: Trastuzumab, Necitumumab, Pembrolizumab, Atezolizumab, Sacituzumab govitecan, Belantamab Mafodotin, Ramucirumab, Elotuzumab, Blinatumomab, Loncastuximab tesirine, and Polatuzumab vedotin.

Furthermore, the macromolecular antibody-based anti-cancer drug includes a monoclonal antibody-based drug, a polyclonal antibody-based drug, and an antibody-drug conjugate, each targeting a specific target.

Furthermore, the recombinant oncolytic virus further includes an antigen encoded by an exogenous gene.

Furthermore, the antigen is one or more selected from a group consisting of: HER2, EGFR, 5T4, B7-H3, TROP2, BCMA, VEGFR-2, SLAMF7, CD19, CD79b, CPC3, and Claudin 18.2.

Furthermore, the recombinant oncolytic virus further includes a cytokine encoded by an exogenous gene.

Furthermore, the cytokine is one or more selected from a group consisting of: IL-12 and IL-18.

In the present application, the recombinant oncolytic virus of the present application can be obtained through virus packaging and virus rescue processes. The specific process may include infecting BSR-T7 cells with the vaccinia virus vTF7-3 expressing T7 RNA polymerase, followed by Lipofectamine-mediated transfection using expression plasmids individually cloning the VSV N, P, and L genes together with a backbone plasmid, thereby obtaining the desired recombinant oncolytic virus.

The present application provides a composition, which includes the recombinant oncolytic virus and the macromolecular antibody-based anti-cancer drug as described above.

In some embodiments, the composition may further include suitable preparations of one or more (pharmaceutically effective) adjuvants, stabilizers, excipients, diluents, solubilizers, surfactants, emulsifiers and/or preservatives. The acceptable ingredients of the composition are preferably non-toxic to the recipient at the dosage and concentration used. The composition of the present application includes, but is not limited to, liquid, frozen and freeze-dried compositions.

In some embodiments, the pharmaceutically acceptable carrier may include any and all solvents, dispersion media, coatings, isotonic agents, and absorption retarders that are compatible with the medication, which are generally safe and non-toxic.

In some embodiments, the composition may be administered parenterally, subcutaneously, intracavity, intraarterially, intravenously, intrathecally and/or intranasally, or injected directly into tissues. For example, the composition may be administered to a patient or a subject by means of infusion or injection. In some embodiments, the administration of the composition may be carried out in different ways, such as intravenous, intraperitoneal, subcutaneous, intramuscular, intradermal or intra-tissue administration. In some embodiments, the composition may be administered incessantly. The incessant (or continuous) administration can be achieved by a small pump system worn by the patient to measure the therapeutic agent flowing into the patient, as described in WO2015/036583.

The present application further provides a use of the above composition in a preparation of a drug for preventing and/or treating a disease and/or a disorder.

The present application will be further described in detail with reference to Preparation Examples and Examples below.

### Preparation Examples

### Preparation Examples 1-10

Preparation Examples 1-10 provide a method for treating a tumor with a combination of a recombinant oncolytic virus and a macromolecular antibody-based anti-cancer drug, respectively.

The method used the recombinant oncolytic virus and the macromolecular antibody-based anti-cancer drug in combination to treat the tumor.

Herein, the recombinant oncolytic virus included an M protein, a G protein, an N protein, a P protein, and an L protein. Herein, the M protein, G protein, N protein, P protein, and L protein were all obtained through site-directed mutations based on the wild-type VSV Indiana MuddSummer strain.

The difference among various Preparation Examples lies in the different mutation sites of the M protein. The mutation sites of M protein are the amino acid sequences as set forth in SEQ ID NO 2 to SEQ ID NO 11, respectively. The G protein includes an amino acid sequence as set forth in SEQ ID NO 13, the N protein includes an amino acid sequence as set forth in SEQ ID NO 15, the P protein includes an amino acid sequence as set forth in SEQ ID NO 17, and the L protein includes an amino acid sequence as set forth in SEQ ID NO 19.

The mutation sites of the proteins and the type of the macromolecular antibody-based anti-cancer drug are shown in Table 1.

The construction method of the recombinant oncolytic viruses provided in the above Preparation Examples is as below:

### (1) Vector Construction

With the pRV-core plasmid (BioVector NTCC Plasmid Vector Strain and Cell Collection Center) as the template, PCR was used to introduce the mutation sites of the M, G, N, P, and L proteins as shown in Table 1, respectively.

Gene fragments containing the aforementioned protein mutation sites and incorporating *Xba*I and *Mlu*I restriction sites were synthesized and used as templates for PCR amplification. The PCR products were then subjected to 1% agarose gel electrophoresis and double digestion with *Xba*I and *Mlu*I, and subsequently recovered by gel extraction using a gel extraction kit. Gene fragments containing the mutation sites for the M, G, N, P, and L proteins were thus obtained, respectively. The RV-core plasmid was double-digested with *Xba*I and *Mlu*I, and the digested pRV-core backbone fragment was recovered by gel extraction using a gel extraction kit.

The above gene fragments containing the mutation sites for the M, G, N, P, and L proteins were individually ligated with the digested pRV-core backbone fragment, followed by transformation and plating. Single colonies were picked for culture and PCR verification to obtain successfully constructed plasmid pRV-core Mut, which was then sent to a sequencing company for sequencing.

**Table 1 Recombinant oncolytic viruses and Macromolecular antibody-based anti-cancer drugs in Preparation Examples 1-10**

| P r e p a r a t i o n E x a m p l e | Recombinant oncolytic virus | | | | | | | | | | | | | | | Macr omol ecular antib ody-b ased anti-c ancer drug |
|---|---|---|---|---|---|---|---|---|---|---|---|---|---|---|---|---|
| | M protein | | | G protein | | | N protein | | | P protein | | | L protein | | | |
| | Mutation sites | Nu mb er of mut atio ns | Sequ ence | Mut atio n sites | Nu mb er of mut atio ns | S eq ue nc e | Mu tati on site s | Nu mb er of mut atio ns | Se qu en ce | Mut atio n site s | Nu mb er of mut atio ns | S eq ue nc e | Mut atio n sites | Nu mb er of mu tati ons | se q u e n c e | |
| 1 | M51R, V221F, S226R | 3 | SEQ ID NO 2 | V53 I, A14 1V, D17 2Y, K21 7E, D23 2G, V33 1A, V37 1E, G43 6D, T43 8S, F45 3L, T47 | 12 | S E Q I D N O 1 3 | I14 V, R1 55 K, S3 53 N | 3 | S E Q ID N O 15 | R50 K, V76 A, D99 E, L12 6S, L14 0S, H15 1Y, I16 8M, K17 0E, Y18 9S, N23 7D | 10 | S E Q I D N O 1 7 | S87 P, I487 T | 2 | S E Q I D N O 1 9 | Trast uzum ab |
| 2 | N32S, N49D, M51R, H54Y, V221F, 225I, S226R | 7 | SEQ ID NO 3 | | | | | | | | | | | | | |
| 3 | N32S, N49D, M51R, H54Y, deletion of the bases encoding leucine at position 111, V221F, V225I, S226R | 8 | SEQ ID NO 4 | | | | | | | | | | | | | |
| 4 | N32S, N49D, M51R, H54Y, L111A, V221F, V225I, S226R | 8 | SEQ ID NO 5 | | | | | | | | | | | | | |
| 5 | G21E, N32S, N49D, M51R, H54Y, V221F, V225I, S226R | 8 | SEQ ID NO | | | | | | | | | | | | | |
| | | | 6 | 1I, Y48 7H | | | | | | | | | | | | |
| 6 | G21E, N32S, M33A, N49D, M51R, H54Y, V221F, V225I, S226R | 9 | SEQ ID NO 7 | | | | | | | | | | | | | |
| 7 | G21E, N32S, M33A, N49D, M51R, H54Y, A133T, V221F, V225I, S226R | 10 | SEQ ID NO 8 | | | | | | | | | | | | | |
| 8 | N32S, M33A, N49D, M51R, H54Y, V221F, V225I, S226R | 8 | SEQ ID NO 9 | | | | | | | | | | | | | |
| 9 | N32S, M33A, N49D, M51R, H54Y, 133T, V221F, 225I, S226R | 9 | SEQ ID NO 10 | | | | | | | | | | | | | |
| 1 0 | N32S, N49D, M51R, H54Y, A133T, V221F, V225I, S226R | 8 | SEQ ID NO 11 | | | | | | | | | | | | | |

### (2) Virus Rescue

The constructed plasmid pRV-core Mut was transfected into BSR-T7 cells (purchased from ATCC, the American Type Culture Collection) through a cell transfection technology by using a calcium phosphate transfection kit (Thermo Fisher Scientific).

Four plasmids, pRV-core Mut, pP, pN, and pL were mixed at a mass ratio of 10:5:4:1, with a total plasmid amount of 5 µg. The plasmids were diluted with 200 µl of opti-MEM medium (Thermo Fisher Scientific), and mixed with 7.5 µl of a transfection reagent, Plus Reagent (Life Technologies), to obtain a premixed solution of transfection plasmids. Herein, pP is a plasmid carrying the rhabdovirus phosphoprotein gene, pN is a plasmid carrying the rhabdovirus nucleoprotein gene, and pL is a plasmid carrying the rhabdovirus polymerase protein gene. The parental vector for all three plasmids (pN, pP, and pL) is pCAGGS (purchased from ATCC).

10 µl of lipofectamine LTX (Thermo Fisher Scientific) was diluted with 200 µl of opti-MEM medium to obtain a mixed LTX solution.

Plasmid transfection was conducted according to the method described in the instructions for lipofectamine LTX. After 6 hours, the BSR-T7 cells were washed twice with PBS, then cultured in DMEM medium (Thermo Fisher Scientific) supplemented with 10% fetal bovine serum for 3 days.

The cell supernatant obtained from the culture of BSR-T7 cells was transferred into Vero cells (Thermo Fisher Scientific), which were then incubated at 37°C for 3 days, and green fluorescence in the cells was observed under a fluorescence microscope to confirm virus rescue. Subsequently, the rescued mutant rhabdovirus library was passaged through Vero cells, and monoclonal virus strains were selected using the established plaque assay system.

(3) Gene sequencing. Viral genomic RNA was extracted using the Trizol kit and reverse-transcribed with random primers. The resulting cDNA was then subjected to PCR using primers designed for the gene sequences of the M protein, G protein, N protein, P protein, and L protein, as well as primers designed for the antigen-encoding gene sequences.

The primer sequences designed for the M protein gene sequence are:
PF: ATGAGTTCCTTAAAGAA;
PR: TCATTTGAAGTGG.

The primer sequences designed for the G protein gene sequence are:
PF: ATGAAGTGCCTTTTGTACTTAG;
PR: TTACTTTCCAAGTCGGTTCATCT.

The primer sequences designed for the N protein gene sequence are:
PF: ATGTCTGTTACAGTCAAGAG;
PR: TCATTTGTCAAATTCTGACTT.

The primer sequences designed for the P protein gene sequence are:
PF: ATGGATAATCTCACAAAAGTTCG;
PR: CTACAGAGAATATTTGACTCTCG.

The primer sequences designed for the L protein gene sequence are:
PF: ATGGAAGTCCACGATTTTGAGA;
PR: TTAATCTCTCCAAGAGTTTTCCT.

The products were recovered after 1% agarose gel electrophoresis and sent to a sequencing company for sequencing. The sequencing results are shown in Table 1.

### Preparation Examples 11-23

Preparation Examples 11-23 provide a method for treating a tumor with a combination of a recombinant oncolytic virus and a macromolecular antibody-based anti-cancer drug, respectively.

The method used the recombinant oncolytic virus and the macromolecular antibody-based anti-cancer drug in combination to treat the tumor.

Herein, the recombinant oncolytic virus included an M protein, a G protein, an N protein, a P protein, an L protein, and an antigen. Herein, the mutation sites of the M protein, G protein, N protein, P protein, and L protein were the same as the corresponding mutation sites in Preparation Example 10.

The difference lies in the type of the antigen and the type of the macromolecular antibody-based anti-cancer drug. The mutation sites of proteins, the type of the antigen, and the type of the macromolecular antibody-based anti-cancer drug are shown in Table 2.

The construction method for the recombinant oncolytic viruses provided in the above Preparation Examples was the same as that in Preparation Example 10, with the difference specifically lying in that:
Between the step (1) of vector construction and the step (2) of virus rescue, a step of inserting an antigen-encoding exogenous gene is further included. Specifically, the plasmid pRV-core Mut obtained in step (1) was double-digested with *Xho*I and *Mlu*I to recover long fragments. The antigen-encoding exogenous gene was synthesized by a gene synthesis company, amplified using appropriate primers, and subjected to double digestion with *Xho*I and *Nhe*I to recover the target gene fragment. The pRV-core Mut and the exogenous gene fragment, both subjected to the above double digestion, were ligated and transformed. Single clones were selected, identified by PCR or enzyme digestion, and then sent to a sequencing company for sequencing to obtain the plasmid pRV-core Mut carrying the exogenous gene. In step (2) of virus rescue, the plasmid pRV-core Mut carrying the exogenous gene was used to transfect BSR-T7 cells.

Step (3) involves the sequencing of various types of antigens. Viral genomic RNA was extracted using the Trizol kit and reverse-transcribed with random primers. The resulting cDNA was then subjected to PCR using primers designed for the antigen-encoding gene sequences.

The primer sequences designed for the antigen-encoding gene sequences are:
HER2 F: ATGGAGCTGGCGGCCTTGTGCC;
HER2 R: TTAGATGAGGATCCCAAAGACCA.
2) EGFR F: ACGCTCGAGATGCGACCCTCCGGGACGG;
EGFR R: TCTGGCTAGCTTACATGAAGAGGCCGAT.
3) 5T4 F: ATGTCTTCTCCCACCTCCTCG;
5T4 R: TCACAAATACAAAACCAGGAG.
4) B7-H3 F: ATGCTGCGTCGGCGGGGCAGC;
B7-H3 R: CACGAAAGCCAGGGCCACCAG.
5) TROP2 F: ATGGCTCGGGGCCCCGGCCTC;
TROP2 R: CTACAAGCTCGGTTCCTTTCT.
6) BCMA F: ATGTTGCAGATGGCTGGGCAG;
BCMA R: TTACCTAGCAGAAATTGATTT.
7) VEGFR-2 F: AGGCGCTGGGAGAAAGAACCG;
VEGFR-2 R: TTAATGCGGCTACTTCCTGCT.
8) SLAMF7 F: ATGGCTGGTTCCCCAACATGC;
SLAMF7 R: TCATAGGCAAATAGCCTTGGT.
9) CD19 (SEQ ID NO 28) F: ACGCTCGAGATGCCACCTCCTCGCCTCC;
CD19 (SEQ ID NO 28) R: TCTGGCTAGCTCATCTTTTCCTCCTCAGG.
10) CD19 (SEQ ID NO 29) F: ATGCCCGAGGAACCTCTAGTG;
CD19 (SEQ ID NO 29) R: TTAAAGATGAAGAATGCCCAC.
11) CD79b F: ATGGCCAGGCTGGCGTTGTCT;
CD79b R: TCACTCCTGGCCTGGGTGCTC.
12) GPC3 F: ATGGCCGGGACCGTGCGCACC;
GPC3 R: TCAGTGCACCAGGAAGAAGAA.
13) Claudin 18.2 F: ACGCTCGAGATGGACCAGTGGAGCACCC;
Claudin 18.2 R: TCTGGCTAGCTTAGGCGATGCACATCATC.
14) VEGF F: ATGACGGACAGACAGACAGAC;
VEGF R: TCACCGCCTCGGCTTGTCACA.
15) HER3 F: ATGAGGGCGAACGACGCTCTG;
HER3 R: GAGAAAAGTGCCGCCCAGCAT.
16) NY-ESO-1 F: ATGCAGGCAGAAGGAAGAGGC;
NY-ESO-1 R: TCATCTTCTCTGTCCGCTAGG.
17) MSLN F: ACGCTCGAGATGGAAGTGGAGAAGACAG;
MSLN R: TCTGGCTAGCTCAGGCCAGGGTGGAGGCT.
18) CD30 F: ATGCGCGTCCTCCTCGCCGCG;
CD30 R: TCACTTTCCAGAGGCAGCTGT.

The products were recovered after 1% agarose gel electrophoresis and sent to a sequencing company for sequencing. The sequencing results are shown in Table 2.

**Table 2 Recombinant oncolytic viruses and Macromolecular antibody-based anti-cancer drugs in Preparation Examples 11-23**

| Preparation Example | Protein | Antigen | | Macromolecular antibody-based anti-cancer drug |
|---|---|---|---|---|
| | Mutation site and Mutated amino acids | Type | Sequence | |
| 11 | M protein: N32S, N49D, M51R, H54Y, A133T, V221F, V225I, and S226R. | HER2 | SEQ ID NO 20 | Trastuzumab |
| 12 | G protein: V53I, A141V, D172Y, K217E, D232G, | EGFR | SEQ ID NO | Necitumumab |
| | V331A, V371E, G436D, T438S, F453L, T471I, and Y487H. | | 21 | |
| 13 | | 5T4 | SEQ ID NO 22 | Anti-5T4 antibody [EPR5529] (ab134162) |
| | N protein: I14V, R155K, and S353N. | | | |
| | P protein: R50K, V76A, D99E, L126S, L140S, H151Y, | | | |
| 14 | I168M, K170E, Y189S, and N237D. | B7-H3 | SEQ ID NO 23 | Anti-CD276 antibody [EPNCIR122] (ab134161) |
| | L protein: S87P and I487T. | | | |
| 15 | | TROP2 | SEQ ID NO 24 | Sacituzumab govitecan |
| 16 | | BCMA | SEQ ID NO 25 | Belantamab Mafodotin |
| 17 | | VEGFR-2 | SEQ ID NO 26 | Ramucirumab |
| 18 | | SLAMF7 | SEQ ID NO 27 | Elotuzumab |
| 19 | | CD19 | SEQ ID NO 28 | Blinatumomab |
| 20 | | CD19 | SEQ ID NO 29 | Loncastuximab tesirine |
| 21 | | CD79b | SEQ ID NO 30 | Polatuzumab vedotin |
| 22 | | GPC3 | SEQ ID NO 31 | Anti-Glypican 3 antibody [SP86] (ab95363) |
| | | GPC3(R-S) | SEQ ID NO 32 | |
| | | GPC3(delete YR) | SEQ ID NO 33 | |
| 23 | | Claudin 18.2 (3TM) | SEQ ID NO 34 | Anti-Claudin18.2 antibody [EPR19202] (ab222512) |
| | | Claudin 18.2 (LINKER) | SEQ ID NO 35 | |

### Preparation Examples 24-49

Preparation Examples 24-49 provide a method for treating a tumor with a combination of a recombinant oncolytic virus and a macromolecular antibody-based anti-cancer drug, respectively.

The method used the recombinant oncolytic virus and the macromolecular antibody-based anti-cancer drug in combination to treat the tumor.

Herein, the above Preparation Examples differ from Preparation Example 11 in that, in addition to the M, G, N, P, and L proteins as well as the antigen, the recombinant oncolytic virus further includes a cytokine. Herein, the mutation sites of the M, G, N, P, and L proteins were the same as the corresponding mutation sites in Preparation Example 10.

The difference lies in the type of the cytokine and the type of the macromolecular antibody-based anti-cancer drug. The mutation sites of proteins, the type of the antigen, the type of the cytokine, and the type of the macromolecular antibody-based anti-cancer drug are shown in Table 3.

The construction method for the recombinant oncolytic viruses provided in the above Preparation Examples was the same as that in Preparation Example 10, with the difference specifically lying in that:
Between the step (1) of vector construction and the step (2) of virus rescue, a step of inserting a cytokine-encoding exogenous gene is further included, specifically with reference to the step of inserting an antigen-encoding exogenous gene. The antigen and the cytokine are inserted between G protein and L protein. The order of insertion can be either inserting the cytokine first, followed by the antigen, or inserting the antigen first, followed by the cytokine. In the present application, the cytokine is inserted first, followed by the antigen.

Step (3) also involves the sequencing of cytokines. Viral genomic RNA was extracted using the Trizol kit and reverse-transcribed with random primers. The resulting cDNA was then subjected to PCR using primers designed for the cytokine gene sequences.

The primer sequences designed for the cytokine-encoding gene sequences are:
IL2 (mut4)F: ATGGCCCCCACAAGCTC,
IL2 (mut4) R: TTAGGTCAGTGTGCTG.
IL2 (mut2)F: ATGGCCCCCACAAGCTC,
IL2 (mut2) R: TTAGCCGGTACAAATCAG.
IL12 F: CCCTCGAGATGTGGCCCCCTGGGT,
IL12 R: CGGCTAGCTTAACTGCAGGGCACAGATG.
GMCSF F: ATGTGGCTGCAGAGCCTGCTG;
GMCSFR: TCACTCCTGGACTGGCTCCCA.

The products were recovered after 1% agarose gel electrophoresis and sent to a sequencing company for sequencing. The sequencing results are shown in Table 3.

**Table 3 Recombinant oncolytic viruses and macromolecular antibody-based anti-cancer drugs in Preparation Examples 24-49**

| Pre paration Example | Protein mutation site | Antigen/Cytokine | | | | Macromolecular antibody-based anti-cancer drug |
|---|---|---|---|---|---|---|
| | | Type | Sequence | Type | Sequence | |
| 4 | M protein: N32S, N49D, M51R, H54Y, A133T, V221F, V225I, and S226R. | HER2 | SEQ ID NO 20 | IL-12 | SEQ ID NO 48, SEQ ID NO 49 | Trastuzumab |
| 25 | | EGFR | SEQ ID NO 21 | | | Necitumumab |
| 26 | | 5T4 | SEQ ID NO 22 | IL-2 | SEQ ID NO 46 | Anti-5T4 antibody [EPR5529] (ab134162) |
| | G protein: V53I, A141V, D172Y, K217E, D232G, V331A, V371E, G436D, T438S, F453L, T471I, and Y487H. | | | | | |
| 27 | | B7-H3 | SEQ ID NO 23 | IL-2 | SEQ ID NO 47 | Anti-CD276 antibody [EPNCIR122] (ab134161) |
| | N protein: I14V, R155K, and S353N. | | | | | |
| 28 | P protein: R50K, V76A, D99E, L126S, L140S, H151Y, I168M, K170E, Y189S, and N237D. | TROP2 | SEQ ID NO 24 | IL-12 | SEQ ID NO 48, SEQ ID NO 49 | Sacituzumab govitecan |
| 29 | | BCMA | SEQ ID NO 25 | | | Belantamab Mafodotin |
| | L protein: S87P and I487T. | | | | | |
| 30 | | VEGFR-2 | SEQ ID NO 26 | GMC SF | SEQ ID NO 50 | Ramucirumab |
| 31 | | SLAMF7 | SEQ ID NO | IL-12 | SEQ ID NO | Elotuzumab |
| | | | 27 | | 48, SEQ ID NO 49 | |
| 32 | | CD19 | SEQ ID NO 28 | | | Blinatumomab |
| 33 | | CD19 | SEQ ID NO 29 | | | Loncastuximab tesirine |
| 34 | | CD79b | SEQ ID NO 30 | | | Polatuzumab vedotin |
| 35 | | GPC3 | SEQ ID NO 31 | | | Anti-Glypican 3 antibody [SP86] (ab95363) |
| | | GPC3(R-S) | SEQ ID NO 32 | | | |
| | | GPC3(delete YR) | SEQ ID NO 33 | | | |
| 36 | | Claudin 18.2 (3TM) | SEQ ID NO 34 | | | Anti-Claudin18.2 antibody [EPR19202] (ab222512) |
| | | Claudin 18.2 (LINKER) | SEQ ID NO 35 | | | |
| 37 | | TROP2 | SEQ ID NO 24 | IL-12 | SEQ ID NO 48, SEQ ID NO 49 | Trastuzumab |
| 38 | | VEGF | SEQ ID NO 36 | | | Necitumumab |
| 39 | | HER3 | SEQ ID NO 37 | IL-2 | SEQ ID NO 46 | Anti-5T4 antibody [EPR5529] (ab134162) |
| 40 | M protein: N32S, N49D, M51R, H54Y, A133T, V221F, V225I, and S226R. | NY-ESO-1 | SEQ ID NO 38 | IL-2 | SEQ ID NO 47 | Anti-CD276 antibody [EPNCIR122] (ab134161) |
| | G protein: V53I, A141V, D172Y, K217E, D232G, V331A, V371E, G436D, T438S, F453L, T471I, and Y487H. | | | | | |
| 41 | | Claudin 18.2 | SEQ ID NO 34 | IL-12 | SEQ ID NO 48, SEQ ID NO 49 | Sacituzumab govitecan |
| 42 | | SLAMF7 | SEQ ID NO 27 | | | Belantamab Mafodotin |
| | N protein: I14V, R155K, and S353N. | | | | | |
| 43 | P protein: R50K, V76A, D99E, L126S, L140S, H151Y, I168M, K170E, Y189S, and N237D. | VEGF | SEQ ID NO 36 | GMC SF | SEQ ID NO 50 | Ramucirumab |
| 44 | | MLSN | SEQ ID NO 39 | IL-12 | SEQ ID NO 48, SEQ ID NO 49 | Elotuzumab |
| | L protein: S87P and I487T. | | | | | |
| 45 | | CD30 | SEQ ID NO 40 | | | Blinatumomab |
| 46 | | CD30 | SEQ ID NO 40 | | | Loncastuximab tesirine |
| 47 | | GPC3 | SEQ ID NO 31 | | | Polatuzumab vedotin |
| 48 | | MLSN | SEQ ID NO 39 | | | Anti-Glypican 3 antibody [SP86] |
| | | | | | | (ab95363) |
| 49 | | HER3 | SEQ ID NO 37 | | | Anti-Claudin18.2 antibody [EPR19202] (ab222512) |

### Preparation Examples 50-82

Preparation Examples 50-82 provide a method for treating a tumor with a recombinant oncolytic virus, respectively.

The recombinant oncolytic viruses used in the above Preparation Examples were the recombinant oncolytic viruses used in Preparation Examples 10, 11-23, 24-36, respectively, specifically as shown in Table 4. The difference lies in the type of antigens expressed by the recombinant oncolytic virus (e.g., Preparation Examples 51-66 and Preparation Examples 67-82) or that the recombinant oncolytic virus does not express any antigens (e.g., Preparation Example 50).

**Table 4 Recombinant oncolytic viruses in Preparation Examples 50-82**

| Preparation Example | Protein mutation site | Antigen | | Cytokine | |
|---|---|---|---|---|---|
| | | Type | Sequence | Type | Sequence |
| 50 | | - | - | - | - |
| 51 | | HER2 | SEQ ID NO 20 | - | - |
| 52 | | EGFR | SEQ ID NO 21 | - | - |
| 53 | | 5T4 | SEQ ID NO 22 | - | - |
| 54 | | B7-H3 | SEQ ID NO 23 | - | - |
| 55 | M protein: N32S, N49D, M51R, H54Y, A133T, V221F, V225I, and S226R. | TROP2 | SEQ ID NO 24 | - | - |
| 56 | G protein: V53I, A141V, D172Y, K217E, D232G, V331A, V371E, G436D, T438S, F453L, T471I, and Y487H. | BCMA | SEQ ID NO 25 | - | - |
| 57 | | VEGFR-2 | SEQ ID NO 26 | - | - |
| | N protein: I14V, R155K, and S353N. | | | | |
| 58 | P protein: R50K, V76A, D99E, L126S, L140S, H151Y, I168M, K170E, Y189S, and N237D. | SLAMF7 | SEQ ID NO 27 | - | - |
| 59 | | CD19 | SEQ ID NO 28 | - | - |
| | L protein: S87P and I487T. | | | | |
| 60 | | CD19 | SEQ ID NO 29 | - | - |
| 61 | | CD79b | SEQ ID NO 30 | - | - |
| 62 | | GPC3 | SEQ ID NO 31 | - | - |
| 63 | | GPC3(R-S) | SEQ ID NO 32 | - | - |
| 64 | | GPC3(delete YR) | SEQ ID NO 33 | - | - |
| 65 | | Claudin 18.2 (3TM) | SEQ ID NO 34 | - | - |
| 66 | | Claudin 18.2 (LINKER) | SEQ ID NO 35 | - | - |
| 67 | | HER2 | SEQ ID NO 20 | IL-12 | SEQ ID NO 48, SEQ ID NO 49 |
| 68 | | EGFR | SEQ ID NO 21 | | |
| 69 | | 5T4 | SEQ ID NO 22 | IL-2 | SEQ ID NO 46 |
| 70 | | B7-H3 | SEQ ID NO 23 | IL-2 | SEQ ID NO 47 |
| 71 | | TROP2 | SEQ ID NO 24 | IL-12 | SEQ ID NO 48, SEQ ID NO 49 |
| 72 | M protein: N32S, N49D, M51R, H54Y, A133T, V221F, V225I, and S226R. | BCMA | SEQ ID NO 25 | | |
| 73 | | VEGFR-2 | SEQ ID NO 26 | GMC SF | SEQ ID NO 50 |
| | G protein: V53I, A141V, D172Y, K217E, D232G, V331A, V371E, G436D, T438S, F453L, T471I, and Y487H. | | | | |
| 74 | | SLAMF7 | SEQ ID NO 27 | IL-12 | SEQ ID NO 48, SEQ ID NO 49 |
| 75 | N protein: I14V, R155K, and S353N. | CD19 | SEQ ID NO 28 | | |
| | P protein: R50K, V76A, D99E, L126S, L140S, H151Y, I168M, K170E, Y189S, and N237D. | | | | |
| 76 | | CD19 | SEQ ID NO 29 | | |
| 77 | L protein: S87P and I487T. | CD79b | SEQ ID NO 30 | | |
| 78 | | GPC3 | SEQ ID NO 31 | | |
| 79 | | GPC3(R-S) | SEQ ID NO 32 | | |
| 80 | | GPC3(delete YR) | SEQ ID NO 33 | | |
| 81 | | Claudin 18.2 (3TM) | SEQ ID NO 34 | | |
| 82 | | Claudin 18.2 (LINKER) | SEQ ID NO 35 | | |
| Note: "-" indicates no insertion. | | | | | |

### Preparation Examples 83-95

Preparation Examples 83-95 provide a method for treating a tumor with a macromolecular antibody-based anti-cancer drug, respectively.

The macromolecular antibody-based anti-cancer drugs in the above Preparation Examples were the macromolecular antibody-based anti-cancer drugs in Preparation Examples 11-23, respectively, specifically as shown in Table 5. The difference lies in the type of the macromolecular antibody-based anti-cancer drug.

**Table 5 Macromolecular antibody-based anti-cancer drugs in Preparation Examples 83-95**

| Preparation Example | Macromolecular antibody-based anti-cancer drug |
|---|---|
| 83 | Trastuzumab |
| 84 | Necitumumab |
| 85 | Anti-5T4 antibody [EPR5529] (ab134162) |
| 86 | Anti-CD276 antibody [EPNCIR122] (ab134161) |
| 87 | Sacituzumab govitecan |
| 88 | Belantamab Mafodotin |
| 89 | Ramucirumab |
| 90 | Elotuzumab |
| 91 | Blinatumomab |
| 92 | Loncastuximab tesirine |
| 93 | Polatuzumab vedotin |
| 94 | Anti-Glypican 3 antibody [SP86] (ab95363) |
| 95 | Anti-Claudin18.2 antibody [EPR19202] (ab222512) |

### Example

In this example, animal experiments were conducted using the methods for treating tumors with a recombinant oncolytic virus and/or a macromolecular antibody-based anti-cancer drug provided in Preparation Examples 1-95, respectively.

Balb/c mice were prepared and inoculated with the murine colon cancer cell line MC38 at a dose of 1 × 10⁶ cells per 0.1 mL per mouse. When the average tumor volume of mice reached 50 mm³, the mice were divided into groups. The day of grouping was designated as Day 0, and drug administration was initiated on the same day. The mice were weighed three times per week and observed daily. During the experiment, changes in body weight (g) and tumor volume (mm³) were recorded.

The mice shall be euthanized if the following situations occur: 1. the tumor volume reaches 2000 mm³; 2. the weight of the mice decreases by more than 20%; 3. ulceration occurs on the tumor surface; 4. the mice become paralyzed, etc.

Herein, the wild-type oncolytic virus and the recombinant oncolytic virus were administered via intratumoral injection (IT) and/or intravenous injection (IV) at a dose of 3e8 PFU per mouse. The administration volume was 2.5 ml/kg for IT and 5 ml/kg for IV. The dosing frequency was once every 2 days for a total of 7 doses (Q2D×7).

The macromolecular antibody-based anti-cancer drugs were administered via intraperitoneal administration (IP), with the dose depending on the type of the macromolecular antibody-based anti-cancer drug. The administration volume was 10 ml/kg, and the dosing frequency was twice a week (BIW, administration on days 0, 3, 7, and 10) for a total of 4 doses.

With regard to the Vehicle Control (Blank Control), (10% DMSO + 40% PEG300 + 5% Tween-80 + 45% saline) was used in place of the macromolecular antibody-based anti-cancer drug, which was administered via intraperitoneal administration (IP) at a dose of N/A. The administration volume was 10 ml/kg and the dosing frequency was twice a week (BIW, administration on days 0, 3, 7, and 10) for a total of 4 doses.

### I. Animal experiments on the macromolecular antibody-based anti-cancer drug Trastuzumab

Animal experiments were conducted using the methods for treating tumors with the recombinant oncolytic virus and/or the macromolecular antibody-based anti-cancer drug provided in Preparation Examples 1-10, 11, 24, 37, 51, 67, 50, and 83, respectively. The specific details are shown in Table 6.

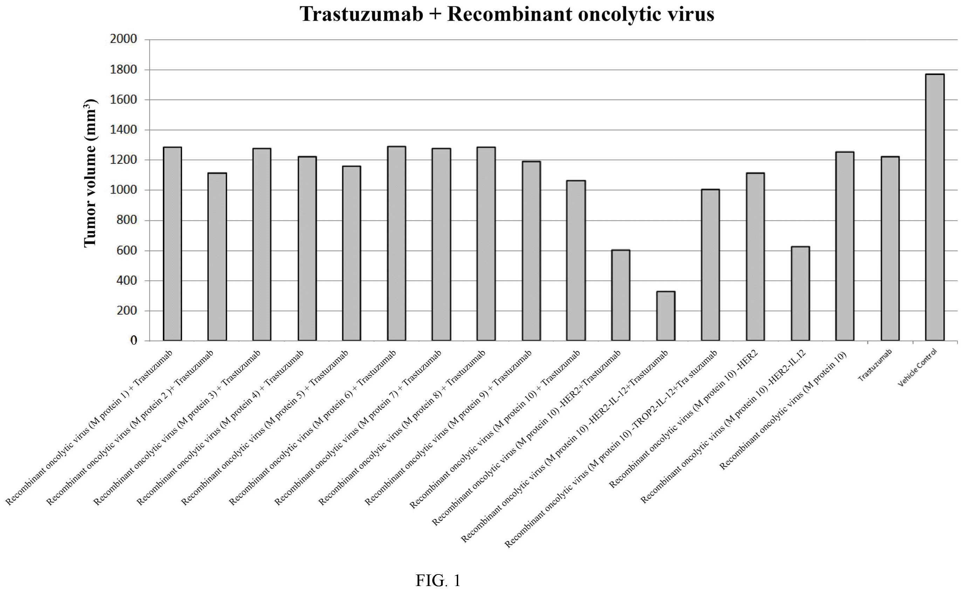

The results are shown in FIG. 1.

**Table 6 Animal experiments on the macromolecular antibody-based anti-cancer drug Trastuzumab**

| Group No. | Corresponding Preparation Example | Corresponding Method | Number of animals | Dose | Dosing Frequency | Route of Administration |
|---|---|---|---|---|---|---|
| 1 | 1 | Recombinant oncolytic virus (M protein 1) + Trastuzumab | 10 | 3e8 PFU per mouse + 54.6 mg/kg | Q2D*7 + BIW | IT + PO |
| 2 | 2 | Recombinant oncolytic virus (M protein 2) + Trastuzumab | 10 | | | |
| 3 | 3 | Recombinant oncolytic virus (M protein 3) + Trastuzumab | 10 | | | |
| 4 | 4 | Recombinant oncolytic virus (M protein 4) + Trastuzumab | 10 | | | |
| 5 | 5 | Recombinant oncolytic virus (M protein 5) + Trastuzumab | 10 | | | |
| 6 | 6 | Recombinant oncolytic virus (M protein 6) + Trastuzumab | 10 | | | |
| 7 | 7 | Recombinant oncolytic virus (M protein 7) + Trastuzumab | 10 | | | |
| 8 | 8 | Recombinant oncolytic virus (M protein 8) + Trastuzumab | 10 | | | |
| 9 | 9 | Recombinant oncolytic virus (M protein 9) + Trastuzumab | 10 | | | |
| 10 | 10 | Recombinant oncolytic virus (M protein 10) + Trastuzumab | 10 | | | |
| 11 | 11 | Recombinant oncolytic virus (M protein 10)-HER2 + Trastuzumab | 10 | | | |
| 12 | 24 | Recombinant oncolytic virus (M protein 10)-HER2-IL-12 + Trastuzumab | 10 | | | |
| 13 | 37 | Recombinant oncolytic virus (M protein 10)-TROP2-IL-12 + Trastuzumab | 10 | | | |
| 14 | 51 | Recombinant oncolytic virus (M protein 10)-HER2 | 10 | 3e8 PFU per mouse | Q2D*7 | IT |
| 15 | 67 | Recombinant oncolytic virus (M protein 10)-HER2-IL-12 | 10 | | | |
| 16 | 50 | Recombinant oncolytic virus (M protein 10) | 10 | | | |
| 17 | 83 | Trastuzumab | 10 | 54.6 mg/kg | BIW | PO |
| 18 | Vehicle Control (Blank Control) | | 10 | N/A | BIW | PO |

### II. Animal experiments on the macromolecular antibody-based anti-cancer drug Necitumumab

Animal experiments were conducted using the methods for treating tumors with the recombinant oncolytic virus and/or the macromolecular antibody-based anti-cancer drug provided in Preparation Examples 12, 25, 38, 52, 68, 50, and 84, respectively. The specific details are shown in Table 7.

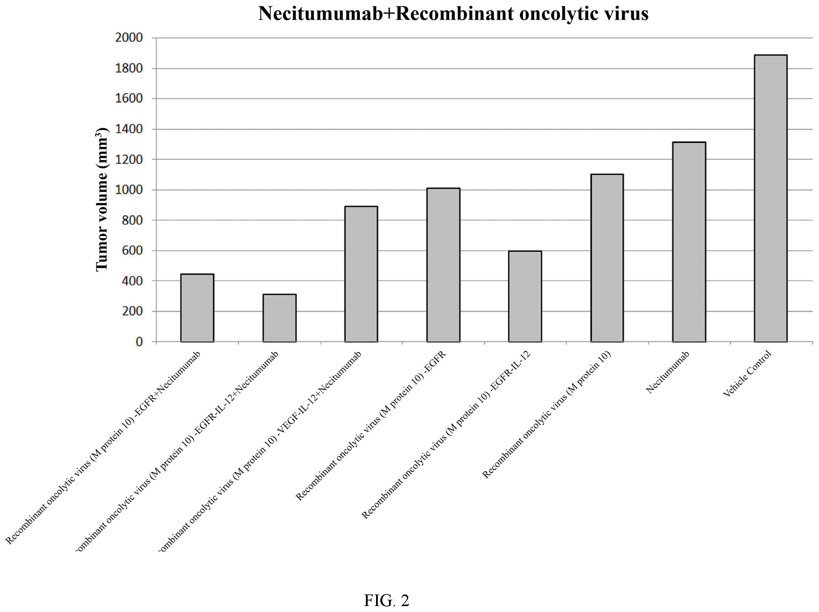

The results are shown in FIG. 2.

**Table 7 Animal experiments on the macromolecular antibody-based anti-cancer drug Necitumumab**

| Group No. | Corresponding Preparation Example | Corresponding Method | Number of animals | Dose | Dosing Frequency | Route of Administration |
|---|---|---|---|---|---|---|
| 1 | 12 | Recombinant oncolytic virus (M protein 10)-EGFR + Necitumumab | 10 | 3e8 PFU per mouse + 104 mg/kg | Q2D*7 + BIW | IT + PO |
| 2 | 25 | Recombinant oncolytic virus (M protein 10)-EGFR-IL-12 + Necitumumab | 10 | | | |
| 3 | 38 | Recombinant oncolytic virus (M protein 10)-VEGF-IL-12 + Necitumumab | 10 | | | |
| 4 | 52 | Recombinant oncolytic virus (M protein 10)-EGFR | 10 | 3e8 PFU per mouse | Q2D*7 | IT |
| 5 | 68 | Recombinant oncolytic virus (M protein 10)-EGFR-IL-12 | 10 | | | |
| 6 | 50 | Recombinant oncolytic virus (M protein 10) | 10 | | | |
| 7 | 84 | Necitumumab | 10 | 104 mg/kg | BIW | PO |
| 8 | Vehicle Control (Blank Control) | | 10 | N/A | BIW | PO |

### III. Animal experiments on the macromolecular antibody-based anti-cancer drug Anti-5T4 antibody

Animal experiments were conducted using the methods for treating tumors with the recombinant oncolytic virus and/or the macromolecular antibody-based anti-cancer drug provided in Preparation Examples 13, 26, 39, 53, 69, 50, and 85, respectively. The specific details are shown in Table 8.

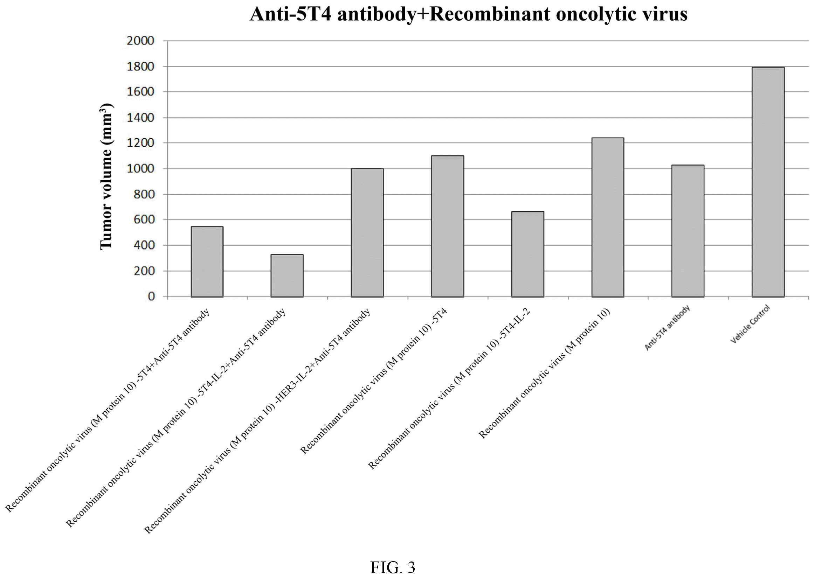

The results are shown in FIG. 3.

**Table 8 Animal experiments on the macromolecular antibody-based anti-cancer drug Anti-5T4 antibody**

| Group No. | Corresponding Preparation Example | Corresponding Method | Number of animals | Dose | Dosing Frequency | Route of Administration |
|---|---|---|---|---|---|---|
| 1 | 13 | Recombinant oncolytic virus (M protein 10)-5T4 + Anti-5T4 antibody | 10 | 3e8 PFU per mouse + 5 mg/kg | Q2D*7 + BIW | IT + PO |
| 2 | 26 | Recombinant oncolytic virus (M protein 10)-5T4-IL-2 + Anti-5T4 antibody | 10 | | | |
| 3 | 39 | Recombinant oncolytic virus (M protein 10)-HER3-IL-2 + Anti-5T4 antibody | 10 | | | |
| 4 | 53 | Recombinant oncolytic virus (M protein 10)-5T4 | 10 | 3e8 PFU per mouse | Q2D*7 | IT |
| 5 | 69 | Recombinant oncolytic virus (M protein 10)-5T4-IL-2 | 10 | | | |
| 6 | 50 | Recombinant oncolytic virus (M protein 10) | 10 | | | |
| 7 | 85 | Anti-5T4 antibody | 10 | 5 mg/kg | BIW | PO |
| 8 | Vehicle Control (Blank Control) | | 10 | N/A | BIW | PO |

### IV. Animal experiments on the macromolecular antibody-based anti-cancer drug Anti-CD276 antibody

Animal experiments were conducted using the methods for treating tumors with the recombinant oncolytic virus and/or the macromolecular antibody-based anti-cancer drug provided in Preparation Examples 14, 27, 40, 54, 70, 50, and 86, respectively. The specific details are shown in Table 9.

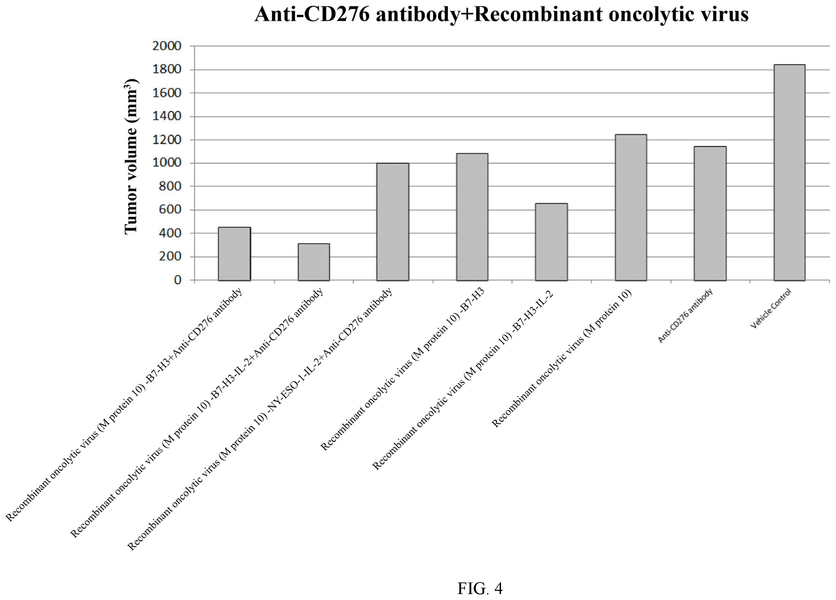

The results are shown in FIG. 4.

**Table 9 Animal experiments on the macromolecular antibody-based anti-cancer drug Anti-CD276 antibody**

| Group No. | Corresponding Preparation Example | Corresponding Method | Number of animals | Dose | Dosing Frequency | Route of Administration |
|---|---|---|---|---|---|---|
| 1 | 14 | Recombinant oncolytic virus (M protein 10)-B7-H3 + Anti-CD276 antibody | 10 | 3e8 PFU per mouse + 10 mg/kg | Q2D*7 + BIW | IT + PO |
| 2 | 27 | Recombinant oncolytic virus (M protein 10)-B7-H3-IL-2 + Anti-CD276 antibody | 10 | | | |
| 3 | 40 | Recombinant oncolytic virus (M protein 10)-NY-ESO-1-IL-2 + Anti-CD276 antibody | 10 | | | |
| 4 | 54 | Recombinant oncolytic virus (M protein 10)-B7-H3 | 10 | 3e8 PFU per mouse | Q2D*7 | IT |
| 5 | 70 | Recombinant oncolytic virus (M protein 10)-B7-H3-IL-2 | 10 | | | |
| 6 | 50 | Recombinant oncolytic virus (M protein 10) | 10 | | | |
| 7 | 86 | Anti-CD276 antibody | 10 | 10 mg/kg | BIW | PO |
| 8 | Vehicle Control (Blank Control) | | 10 | N/A | BIW | PO |

### V. Animal experiments on the macromolecular antibody-based anti-cancer drug Sacituzumab govitecan

Animal experiments were conducted using the methods for treating tumors with the recombinant oncolytic virus and/or the macromolecular antibody-based anti-cancer drug provided in Preparation Examples 15, 28, 41, 55, 71, 50, and 87, respectively. The specific details are shown in Table 10.

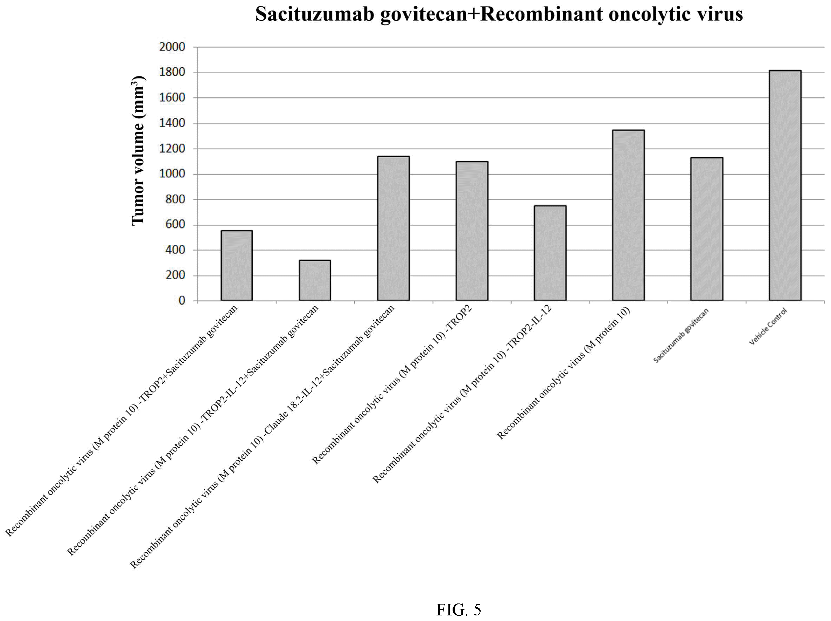

The results are shown in FIG. 5.

**Table 10 Animal experiments on the macromolecular antibody-based anti-cancer drug Sacituzumab govitecan**

| Group No. | Corresponding Preparation Example | Corresponding Method | Number of animals | Dose | Dosing Frequency | Route of Administration |
|---|---|---|---|---|---|---|
| 1 | 15 | Recombinant oncolytic virus (M protein 10)-TROP2 + Sacituzumab govitecan | 10 | 3e8 PFU per mouse + 90 mg/kg | Q2D*7 + BIW | IT + PO |
| 2 | 28 | Recombinant oncolytic virus (M protein 10)-TROP2-IL-12 + Sacituzumab govitecan | 10 | | | |
| 3 | 41 | Recombinant oncolytic virus (M protein 10)-Claudin 18.2-IL-12 + Sacituzumab govitecan | 10 | | | |
| 4 | 55 | Recombinant oncolytic virus (M protein 10)-TROP2 | 10 | 3e8 PFU per mouse | Q2D*7 | IT |
| 5 | 71 | Recombinant oncolytic virus (M protein 10)-TROP2-IL-12 | 10 | | | |
| 6 | 50 | Recombinant oncolytic virus (M protein 10) | 10 | | | |
| 7 | 87 | Sacituzumab govitecan | 10 | 90 mg/kg | BIW | PO |
| 8 | Vehicle Control (Blank Control) | | 10 | N/A | BIW | PO |

### VI. Animal experiments on the macromolecular antibody-based anti-cancer drug Belantamab Mafodotin

Animal experiments were conducted using the methods for treating tumors with the recombinant oncolytic virus and/or the macromolecular antibody-based anti-cancer drug provided in Preparation Examples 16, 29, 42, 56, 72, 50, and 88, respectively. The specific details are shown in Table 11.

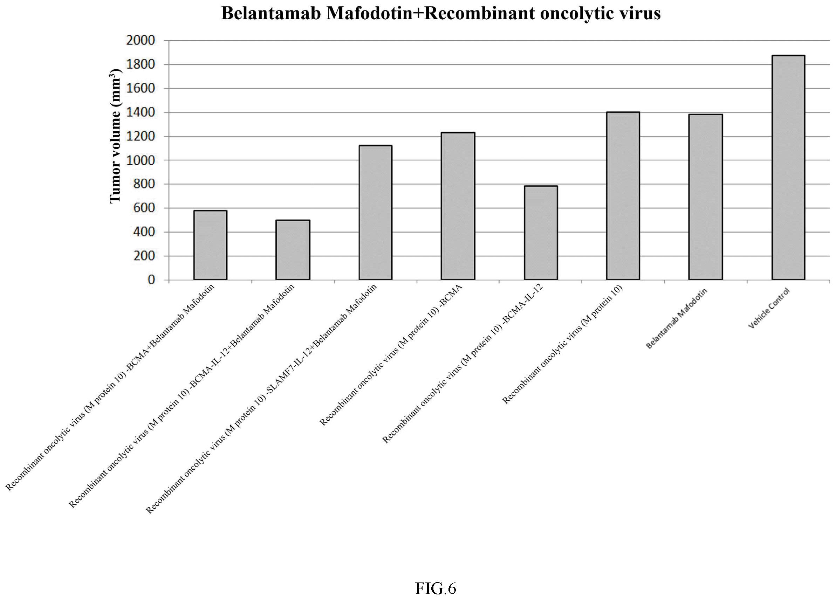

The results are shown in FIG. 6.

**Table 11 Animal experiments on the macromolecular antibody-based anti-cancer drug Belantamab Mafodotin**

| Group No. | Corresponding Preparation Example | Corresponding Method | Number of animals | Dose | Dosing Frequency | Route of Administration |
|---|---|---|---|---|---|---|
| 1 | 16 | Recombinant oncolytic virus (M protein 10)-BCMA + Belantamab Mafodotin | 10 | 3e8 PFU per mouse + 17.29 mg/kg | Q2D*7 + BIW | IT + PO |
| 2 | 29 | Recombinant oncolytic virus (M protein 10)-BCMA-IL-12 + Belantamab Mafodotin | 10 | | | |
| 3 | 42 | Recombinant oncolytic virus (M protein 10)-SLAMF7-IL-12 + Belantamab Mafodotin | 10 | | | |
| 4 | 56 | Recombinant oncolytic virus (M protein 10)-BCMA | 10 | 3e8 PFU per mouse | Q2D*7 | IT |
| 5 | 72 | Recombinant oncolytic virus (M protein 10)-BCMA-IL-12 | 10 | | | |
| 6 | 50 | Recombinant oncolytic virus (M protein 10) | 10 | | | |
| 7 | 88 | Belantamab Mafodotin | 10 | 17.29 mg/kg | BIW | PO |
| 8 | Vehicle Control (Blank Control) | | 10 | N/A | BIW | PO |

### VII. Animal experiments on the macromolecular antibody-based anti-cancer drug Ramucirumab

Animal experiments were conducted using the methods for treating tumors with the recombinant oncolytic virus and/or the macromolecular antibody-based anti-cancer drug provided in Preparation Examples 17, 30, 43, 57, 73, 50, and 89, respectively. The specific details are shown in Table 12.

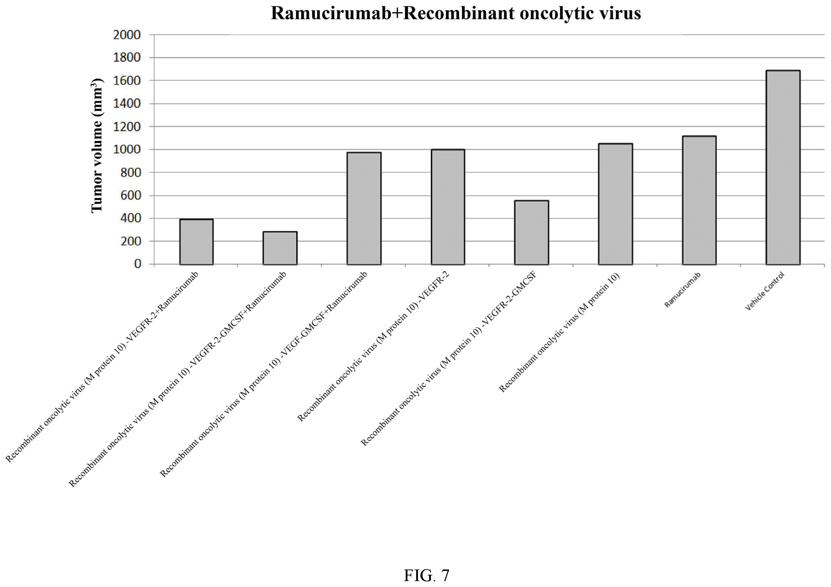

The results are shown in FIG. 7.

**Table 12 Animal experiments on the macromolecular antibody-based anti-cancer drug Ramucirumab**

| Group No. | Corresponding Preparation Example | Corresponding Method | Number of animals | Dose | Dosing Frequency | Route of Administration |
|---|---|---|---|---|---|---|
| 1 | 17 | Recombinant oncolytic virus (M protein 10)-VEGFR-2 + Ramucirumab | 10 | 3e8 PFU per mouse + 91 mg/kg | Q2D*7 + BIW | IT + PO |
| 2 | 30 | Recombinant oncolytic virus (M protein 10)-VEGFR-2-GMCSF + Ramucirumab | 10 | | | |
| 3 | 43 | Recombinant oncolytic virus (M protein 10)-VEGF-GMCSF + Ramucirumab | 10 | | | |
| 4 | 57 | Recombinant oncolytic virus (M protein 10)-VEGFR-2 | 10 | 3e8 PFU per mouse | Q2D*7 | IT |
| 5 | 73 | Recombinant oncolytic virus (M protein 10)-VEGFR-2-GMCSF | 10 | | | |
| 6 | 50 | Recombinant oncolytic virus (M protein 10) | 10 | | | |
| 7 | 89 | Ramucirumab | 10 | 91 mg/kg | BIW | PO |
| 8 | Vehicle Control (Blank Control) | | 10 | N/A | BIW | PO |

### VIII. Animal experiments on the macromolecular antibody-based anti-cancer drug Elotuzumab

Animal experiments were conducted using the methods for treating tumors with the recombinant oncolytic virus and/or the macromolecular antibody-based anti-cancer drug provided in Preparation Examples 18, 31, 44, 58, 74, 50, and 90, respectively. The specific details are shown in Table 13.

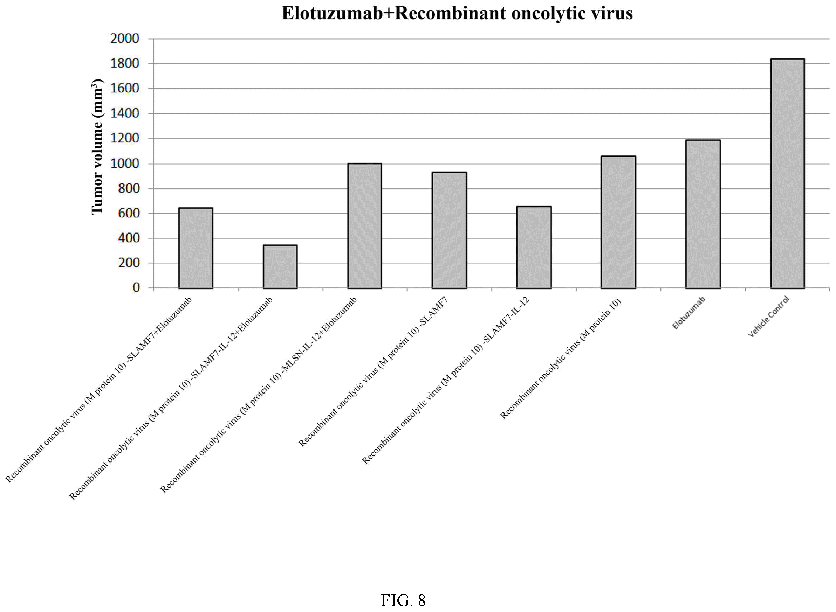

The results are shown in FIG. 8.

**Table 13 Animal experiments on the macromolecular antibody-based anti-cancer drug Elotuzumab**

| Group No. | Corresponding Preparation Example | Corresponding Method | Number of animals | Dose | Dosing Frequency | Route of Administration |
|---|---|---|---|---|---|---|
| 1 | 18 | Recombinant oncolytic virus (M protein 10)-SLAMF7 + Elotuzumab | 10 | 3e8 PFU per mouse + 90 mg/kg | Q2D*7 + BIW | IT + PO |
| 2 | 31 | Recombinant oncolytic virus (M protein 10)-SLAMF7-IL-12 + Elotuzumab | 10 | | | |
| 3 | 44 | Recombinant oncolytic virus (M protein 10)-MLSN-IL-12 + Elotuzumab | 10 | | | |
| 4 | 58 | Recombinant oncolytic virus (M protein 10)-SLAMF7 | 10 | 3e8 PFU per mouse | Q2D*7 | IT |
| 5 | 74 | Recombinant oncolytic virus (M protein 10)-SLAMF7-IL-12 | 10 | | | |
| 6 | 50 | Recombinant oncolytic virus (M protein 10) | 10 | | | |
| 7 | 90 | Elotuzumab | 10 | 90 mg/kg | BIW | PO |
| 8 | Vehicle Control (Blank Control) | | 10 | N/A | BIW | PO |

### IX. Animal experiments on the macromolecular antibody-based anti-cancer drug Blinatumomab

Animal experiments were conducted using the methods for treating tumors with the recombinant oncolytic virus and/or the macromolecular antibody-based anti-cancer drug provided in Preparation Examples 19, 32, 45, 59, 75, 50, and 91, respectively. The specific details are shown in Table 14.

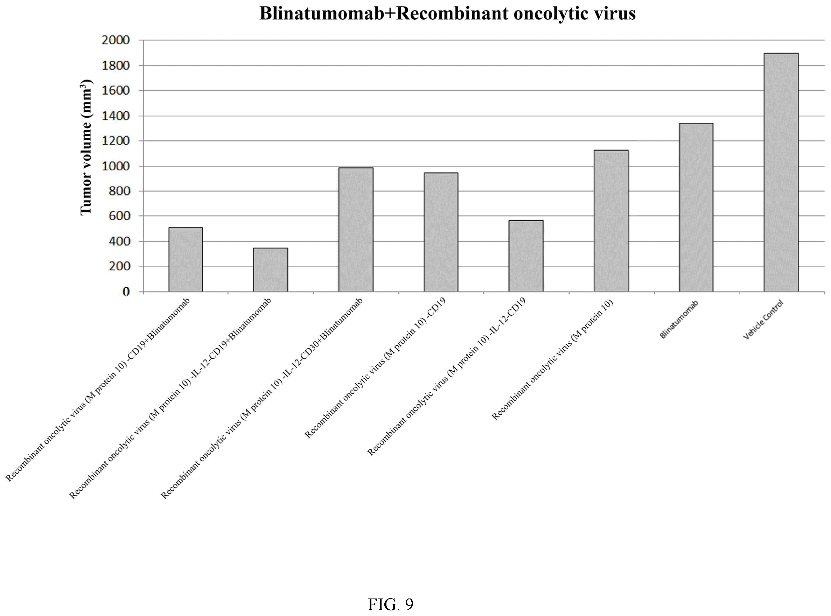

The results are shown in FIG. 9.

**Table 14 Animal experiments on the macromolecular antibody-based anti-cancer drug Blinatumomab**

| Group No. | Corresponding Preparation Example | Corresponding Method | Number of animals | Dose | Dosing Frequency | Route of Administration |
|---|---|---|---|---|---|---|
| 1 | 19 | Recombinant oncolytic virus (M protein 10)-CD19 + Blinatumomab | 10 | 3e8 PFU per mouse + 1.365 mg/kg | Q2D*7 + BIW | IT + PO |
| 2 | 32 | Recombinant oncolytic virus (M protein 10)-IL-12-CD19 + Blinatumomab | 10 | | | |
| 3 | 45 | Recombinant oncolytic virus (M protein 10)-IL-12- CD30 + Blinatumomab | 10 | | | |
| 4 | 59 | Recombinant oncolytic virus (M protein 10)-CD19 | 10 | 3e8 PFU per | Q2D*7 | IT |
| 5 | 75 | Recombinant oncolytic virus | 10 | | | |
| | | (M protein 10)-IL-12-CD19 | | mouse | | |
| 6 | 50 | Recombinant oncolytic virus (M protein 10) | 10 | | | |
| 7 | 91 | Blinatumomab | 10 | 1.365 mg/kg | BIW | PO |
| 8 | Vehicle Control (Blank Control) | | 10 | N/A | BIW | PO |

### X. Animal experiments on the macromolecular antibody-based anti-cancer drug Loncastuximab tesirine

Animal experiments were conducted using the methods for treating tumors with the recombinant oncolytic virus and/or the macromolecular antibody-based anti-cancer drug provided in Preparation Examples 20, 33, 46, 60, 76, 50, and 92, respectively. The specific details are shown in Table 15.

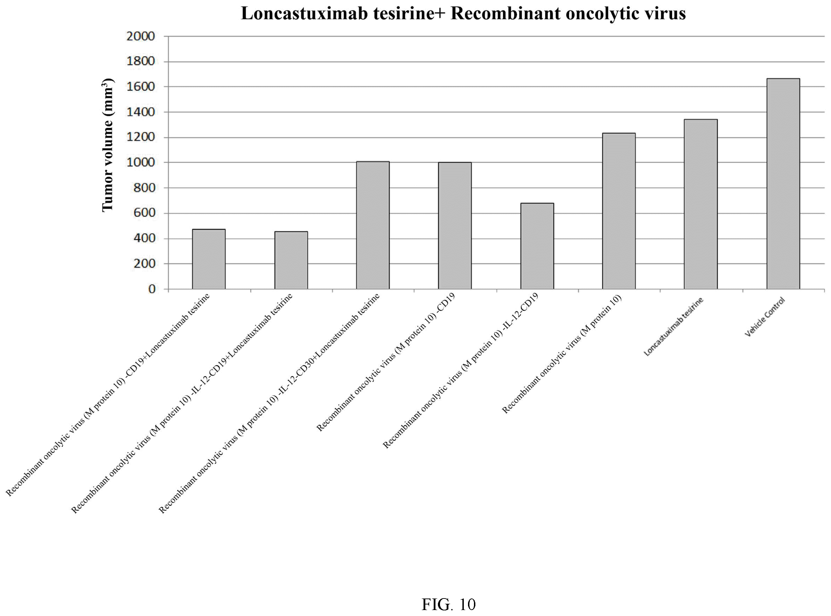

The results are shown in FIG. 10.

**Table 15 Animal experiments on the macromolecular antibody-based anti-cancer drug Loncastuximab tesirine**

| Group No. | Corresponding Preparation Example | Corresponding Method | Number of animals | Dose | Dosing Frequency | Route of Administration |
|---|---|---|---|---|---|---|
| 1 | 20 | Recombinant oncolytic virus (M protein 10)-CD19 + Loncastuximab tesirine | 10 | 3e8 PFU per mouse + 1.365 mg/kg | Q2D*7 + BIW | IT + PO |
| 2 | 33 | Recombinant oncolytic virus (M protein 10)-IL-12-CD19 + Loncastuximab tesirine | 10 | | | |
| 3 | 46 | Recombinant oncolytic virus (M protein 10)-IL-12-CD30 + Loncastuximab tesirine | 10 | | | |
| 4 | 60 | Recombinant oncolytic virus (M protein 10)-CD19 | 10 | 3e8 PFU per mouse | Q2D*7 | IT |
| 5 | 76 | Recombinant oncolytic virus (M protein 10)-IL-12-CD19 | 10 | | | |
| 6 | 50 | Recombinant oncolytic virus (M protein 10) | 10 | | | |
| 7 | 92 | Loncastuximab tesirine | 10 | 1.365 mg/kg | BIW | PO |
| 8 | Vehicle Control (Blank Control) | | 10 | N/A | BIW | PO |

### XI. Animal experiments on the macromolecular anti-cancer drug Polatuzumab vedotin

Animal experiments were conducted using the methods for treating tumors with the recombinant oncolytic virus and/or the macromolecular antibody-based anti-cancer drug provided in Preparation Examples 21, 34, 47, 61, 77, 50, and 93, respectively. The specific details are shown in Table 16.

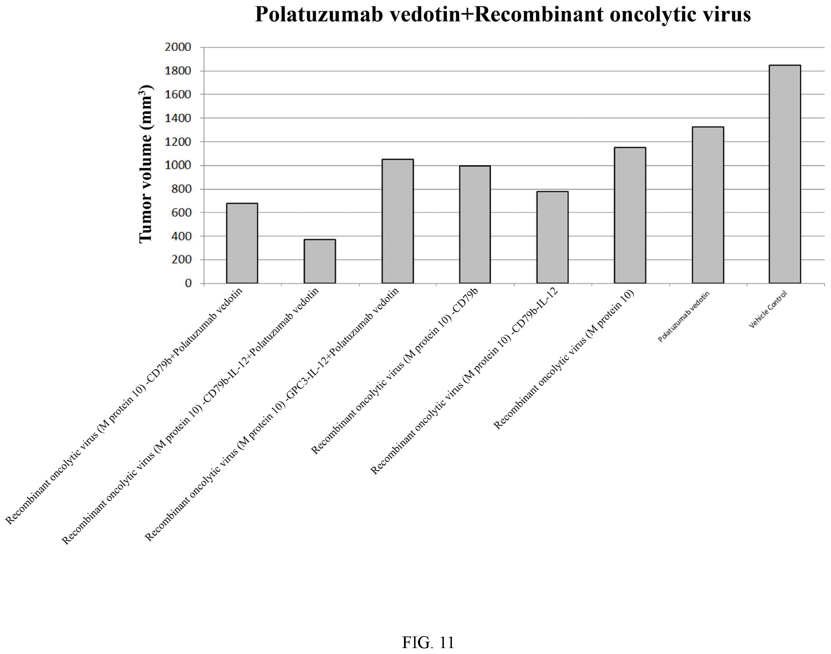

The results are shown in FIG. 11.

**Table 16 Animal experiments on the macromolecular antibody-based anti-cancer drug Polatuzumab vedotin**

| Group No. | Corresponding Preparation Example | Corresponding Method | Number of animals | Dose | Dosing Frequency | Route of Administration |
|---|---|---|---|---|---|---|
| 1 | 21 | Recombinant oncolytic virus (M protein 10)-CD79b + Polatuzumab vedotin | 10 | 3e8 PFU per mouse + 1.8 | Q2D*7 + BIW | IT + PO |
| 2 | 34 | Recombinant oncolytic virus (M protein 10)-CD79b-IL-12 + Polatuzumab vedotin | 10 | mg/kg | | |
| 3 | 47 | Recombinant oncolytic virus (M protein 10)-GPC3-IL-12 + Polatuzumab vedotin | 10 | | | |
| 4 | 61 | Recombinant oncolytic virus (M protein 10)-CD79b | 10 | 3e8 PFU per mouse | Q2D*7 | IT |
| 5 | 77 | Recombinant oncolytic virus (M protein 10)-CD79b-IL-12 | 10 | | | |
| 6 | 50 | Recombinant oncolytic virus (M protein 10) | 10 | | | |
| 7 | 93 | Polatuzumab vedotin | 10 | 1.8 mg/kg | BIW | PO |
| 8 | Vehicle Control (Blank Control) | | 10 | N/A | BIW | PO |

### XII. Animal experiments on the macromolecular antibody-based anti-cancer drug Anti-Glypican 3 antibody

Animal experiments were conducted using the methods for treating tumors with the recombinant oncolytic virus and/or the macromolecular antibody-based anti-cancer drug provided in Preparation Examples 22, 35, 48, 62-64, 78-80, 50, and 94, respectively. The specific details are shown in Table 17.

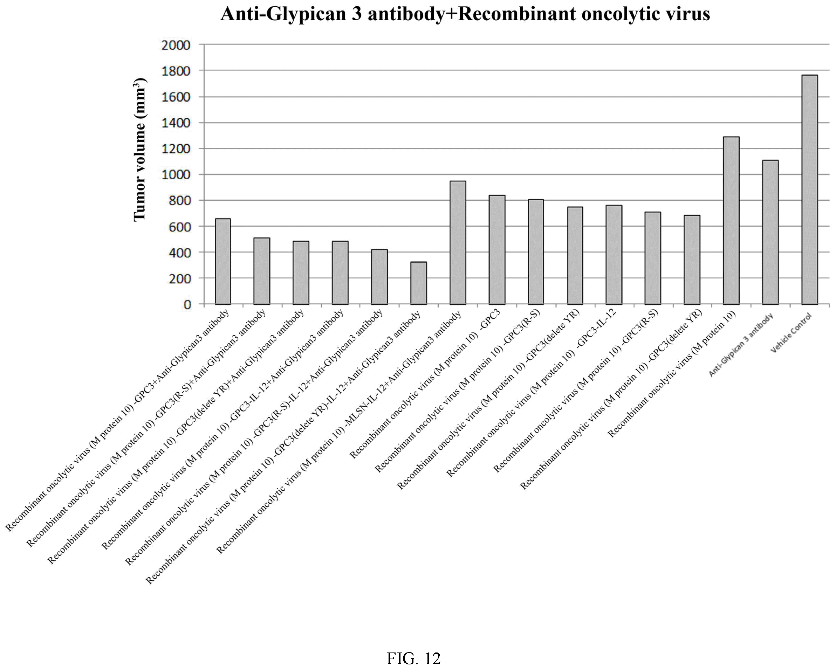

The results are shown in FIG. 12.

**Table 17 Animal experiments on the macromolecular antibody-based anti-cancer drug Anti-Glypican 3 antibody**

| Group No. | Corresponding Preparation Example | Corresponding Method | Number of animals | Dose | Dosing Frequency | Route of Administration |
|---|---|---|---|---|---|---|
| 1 | 22 | Recombinant oncolytic virus (M protein 10)-GPC3 + Anti-Glypican 3 antibody | 10 | | | |
| 2 | | Recombinant oncolytic virus (M protein 10)- GPC3(R-S) + Anti-Glypican 3 antibody | 10 | | | |
| 3 | | Recombinant oncolytic virus (M protein 10)- GPC3(delete YR) + Anti-Glypican 3 antibody | 10 | 3e8 PFU per mouse + 1 mg/kg | Q2D*7 + BIW | IT + PO |
| 4 | 35 | Recombinant oncolytic virus (M protein 10)-GPC3-IL-12 + Anti-Glypican 3 antibody | 10 | | | |
| 5 | | Recombinant oncolytic virus (M protein 10)-GPC3(R-S)-IL-12 + Anti-Glypican 3 antibody | 10 | | | |
| 6 | | Recombinant oncolytic virus (M protein 10)- GPC3(delete YR)-IL-12 + Anti-Glypican 3 antibody | 10 | | | |
| 7 | 48 | Recombinant oncolytic virus | 10 | | | |
| | | (M protein 10)-MLSN-IL-12 + Anti-Glypican 3 antibody | | | | |
| 8 | 62 | Recombinant oncolytic virus (M protein 10)-GPC3 | 10 | 3e8 PFU per mouse | Q2D*7 | IT |
| 9 | 63 | Recombinant oncolytic virus (M protein 10)- GPC3(R-S) | 10 | | | |
| 10 | 64 | Recombinant oncolytic virus (M protein 10)- GPC3(delete YR) | 10 | | | |
| 11 | 78 | Recombinant oncolytic virus (M protein 10)-GPC3-IL-12 | 10 | | | |
| 12 | 79 | Recombinant oncolytic virus (M protein 10)- GPC3(R-S) | 10 | | | |
| 13 | 80 | Recombinant oncolytic virus (M protein 10)- GPC3(delete YR) | 10 | | | |
| 14 | 50 | Recombinant oncolytic virus (M protein 10) | 10 | | | |
| 15 | 94 | Anti-Glypican 3 antibody | 10 | 1 mg/kg | BIW | PO |
| 16 | Vehicle Control (Blank Control) | | 10 | N/A | BIW | PO |

### XIII. Animal experiments on the macromolecular anti-cancer drug Anti-Claudin18.2 antibody

Animal experiments were conducted using the methods for treating tumors with the recombinant oncolytic virus and/or the macromolecular antibody-based anti-cancer drug provided in Preparation Examples 23, 36, 49, 65-66, 81-82, 50, and 95, respectively. The specific details are shown in Table 18.

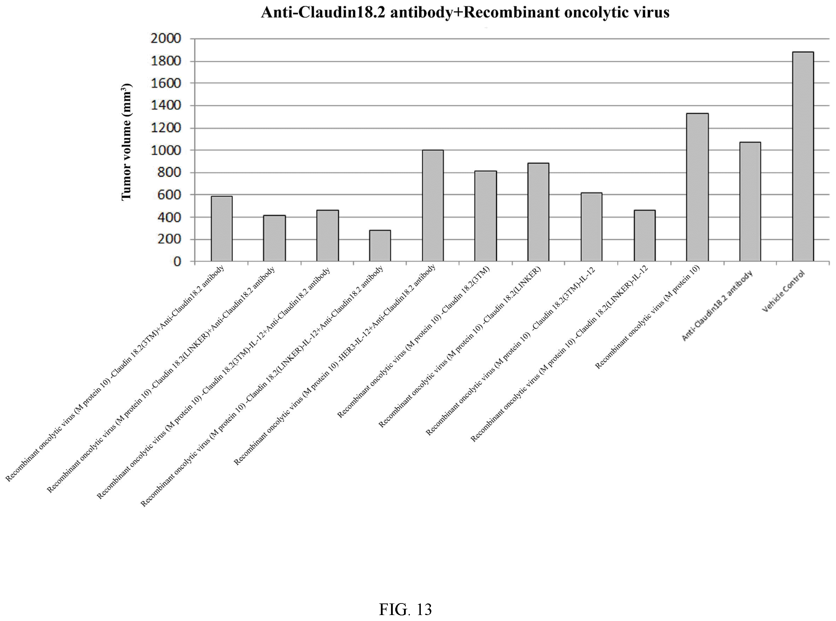

The results are shown in FIG. 13.

**Table 18 Animal experiments on the macromolecular antibody-based anti-cancer drug Anti-Claudin18.2 antibody**

| Group No. | Corresponding Preparation Example | Corresponding Method | Number of animals | Dose | Dosing Frequency | Route of Administration |
|---|---|---|---|---|---|---|
| 1 | 23 | Recombinant oncolytic virus (M protein 10)-Claudin 18.2 (3TM) + Anti-Claudin18.2 antibody | 10 | | | |
| 2 | | Recombinant oncolytic virus (M protein 10)-Claudin 18.2 (LINKER) + Anti-Claudin18.2 antibody | 10 | 3e8 PFU per mouse + 5 mg/kg | Q2D*7 + BIW | IT + PO |
| 3 | 36 | Recombinant oncolytic virus (M protein 10)-Claudin 18.2 (3TM)-IL-12 + Anti-Claudin18.2 antibody | 10 | | | |
| 4 | | Recombinant oncolytic virus (M protein 10)-Claudin 18.2 (LINKER)-IL-12 + | 10 | | | |
| | | Anti-Claudin18.2 antibody | | | | |
| 5 | 49 | Recombinant oncolytic virus (M protein 10)-HER3-IL-12 + Anti-Claudin18.2 antibody | 10 | | | |
| 6 | 65 | Recombinant oncolytic virus (M protein 10)-Claudin 18.2 (3TM) | 10 | 3e8 PFU per mouse | Q2D*7 | IT |
| 7 | 66 | Recombinant oncolytic virus (M protein 10)-Claudin 18.2 (LINKER) | 10 | | | |
| 8 | 81 | Recombinant oncolytic virus (M protein 10)-Claudin 18.2 (3TM)-IL-12 | 10 | | | |
| 9 | 82 | Recombinant oncolytic virus (M protein 10)-Claudin 18.2 (LINKER)-IL-12 | 10 | | | |
| 10 | 50 | Recombinant oncolytic virus (M protein 10) | 10 | | | |
| 11 | 95 | Anti-Claudin18.2 antibody | 10 | 5 mg/kg | BIW | PO |
| 12 | Vehicle Control (Blank Control) | | 10 | N/A | BIW | PO |

The results are shown in FIGs. 1-13.

As can be seen from the above figures, the method for treating a tumor with a combination of a recombinant oncolytic virus and a macromolecular antibody-based anti-cancer drug provided in the present application exhibits a significant inhibitory effect on tumor growth. In addition, the test results of tumor treatment using a recombinant oncolytic virus directly combined with a macromolecular antibody-based anti-cancer drug are superior to those of a recombinant oncolytic virus monotherapy of tumor or a macromolecular antibody-based anti-cancer drug monotherapy of tumor. The test results of tumor treatment using a recombinant oncolytic virus expressing an antigen or an antigen fragment combined with a macromolecular antibody-based anti-cancer drug targeting the antigen or antigen fragment are superior to the test results of tumor treatment using a recombinant oncolytic virus directly combined with a macromolecular antibody-based anti-cancer drug or the test results of tumor treatment directly using a recombinant oncolytic virus expressing an antigen or an antigen fragment. Therefore, the method for treating a tumor with a combination of a recombinant oncolytic virus and a macromolecular antibody-based anti-cancer drug provided in the present application further effectively improves the therapeutic effects on tumor cells.

Meanwhile, in the method for treating a tumor with a combination of a recombinant oncolytic virus and a macromolecular antibody-based anti-cancer drug in the present application, the test results were superior when the recombinant oncolytic virus contains a target that is specifically targeted by the macromolecular antibody-based anti-cancer drug to those obtained when the recombinant oncolytic virus does not contain a target that is specifically targeted by the macromolecular antibody-based anti-cancer drug. Furthermore, the test results obtained when the recombinant oncolytic virus contains a target that is specifically targeted by the macromolecular antibody-based anti-cancer drug and the recombinant oncolytic virus expresses a cytokine were superior to those obtained when the recombinant oncolytic virus contains a target that is specifically targeted by the macromolecular antibody-based anti-cancer drug but the recombinant oncolytic virus does not express any cytokines.

As can be seen from the above test results, the method for treating a tumor with a combination of a recombinant oncolytic virus and a macromolecular antibody-based anti-cancer drug provided in the present application exhibits good inhibitory effects on the growth of tumor cells. It can be inferred that the method for treating a tumor with the combination of a recombinant oncolytic virus and a macromolecular antibody-based anti-cancer drug provided in the present application also exhibits favorable inhibitory effects on other cancer cells, proposing broad clinical application prospects.
This specific example is merely illustrative of the present application and is not intended to limit the present application. After reading this specification, those skilled in the art may make modifications to the embodiments without creative contribution as desired. However, all such modifications shall be protected by patent law as long as they fall within the scope of the claims of the present application.

## Claims

1. A method for treating a tumor with a combination of a recombinant oncolytic virus and a macromolecular antibody-based anti-cancer drug, **characterized by** using the macromolecular antibody-based anti-cancer drug and the recombinant oncolytic virus in combination to treat the tumor;
the macromolecular antibody-based anti-cancer drug comprises a macromolecular antibody-based anti-cancer drug targeting HER2, a macromolecular antibody-based anti-cancer drug targeting EGFR, a macromolecular antibody-based anti-cancer drug targeting PD-1, a macromolecular antibody-based anti-cancer drug targeting PD-L1, a macromolecular antibody-based anti-cancer drug targeting TROP2, a macromolecular antibody-based anti-cancer drug targeting BCMA, a macromolecular antibody-based anti-cancer drug targeting VEGFR-2, a macromolecular antibody-based anti-cancer drug targeting SLAMF7, a macromolecular antibody-based anti-cancer drug targeting CD3, a macromolecular antibody-based anti-cancer drug targeting CD19, a macromolecular antibody-based anti-cancer drug targeting CD20, a macromolecular antibody-based anti-cancer drug targeting CD22, a macromolecular antibody-based anti-cancer drug targeting CD30, a macromolecular antibody-based anti-cancer drug targeting CD33, a macromolecular antibody-based anti-cancer drug targeting CD38, a macromolecular antibody-based anti-cancer drug targeting CD52, a macromolecular antibody-based anti-cancer drug targeting CD79b, a macromolecular antibody-based anti-cancer drug targeting Met, a macromolecular antibody-based anti-cancer drug targeting GD2, a macromolecular antibody-based anti-cancer drug targeting VEGF, a macromolecular antibody-based anti-cancer drug targeting PDGFR-α, a macromolecular antibody-based anti-cancer drug targeting CTLA-4, a macromolecular antibody-based anti-cancer drug targeting RANKL, a macromolecular antibody-based anti-cancer drug targeting FRa, a macromolecular antibody-based anti-cancer drug targeting TF, a macromolecular antibody-based anti-cancer drug targeting IL6, a macromolecular antibody-based anti-cancer drug targeting GPRC5D, a macromolecular antibody-based anti-cancer drug targeting TNF-α, a macromolecular antibody-based anti-cancer drug targeting EPCAM, a macromolecular antibody-based anti-cancer drug targeting CD24, a macromolecular antibody-based anti-cancer drug targeting 5T4, a macromolecular antibody-based anti-cancer drug targeting B7-H3, a macromolecular antibody-based anti-cancer drug targeting GPC3, and a macromolecular antibody-based anti-cancer drug targeting Claudin 18.2;
the recombinant oncolytic virus comprises an M protein, a G protein, an N protein, a P protein, and an L protein;
compared with an amino acid sequence as set forth in SEQ ID NO 1,
an site mutation of the M protein comprises one or more of M51R, V221F, or S226R;
alternatively, the site mutation of the M protein comprises one or more of N32S, N49D, M51R, H54Y, V221F, V225I, or S226R;
alternatively, the site mutation of the M protein comprises one or more of N32S, N49D, M51R, H54Y, knocking out of nucleotide bases encoding leucine at position 111, V221F, V225I, or S226R;
alternatively, the site mutation of the M protein comprises one or more of N32S, N49D, M51R, H54Y, L111A, V221F, V225I, or S226R;
alternatively, the site mutation of the M protein comprises one or more of G21E, N32S, N49D, M51R, H54Y, V221F, V225I, or S226R;
alternatively, the site mutation of the M protein comprises one or more of G21E, N32S, M33A, N49D, M51R, H54Y, V221F, V225I, or S226R;
alternatively, the site mutation of the M protein comprises one or more of G21E, N32S, M33A, N49D, M51R, H54Y, A133T, V221F, V225I, or S226R;
alternatively, the site mutation of the M protein comprises one or more of N32S, M33A, N49D, M51R, H54Y, V221F, V225I, or S226R;
alternatively, the site mutation of the M protein comprises one or more of N32S, M33A, N49D, M51R, H54Y, A133T, V221F, V225I, or S226R;
alternatively, the site mutation of the M protein comprises any one or more of N32S, N49D, M51R, H54Y, A133T, V221F, V225I, or S226R;
compared with an amino acid sequence as set forth in SEQ ID NO 12, an site mutation of the G protein comprises one or more of V53I, A141V, D172Y, K217E, D232G, V331A, V371E, G436D, T438S, F453L, T471I, or Y487H;
compared with the amino acid sequence as set forth in SEQ ID NO 14, an site mutation of the N protein comprises one or more of I14V, R155K, or S353N;
compared with the amino acid sequence as set forth in SEQ ID NO 16, an site mutation of the P protein comprises one or more of R50K, V76A, D99E, L126S, L140S, H151Y, I168M, K170E, Y189S, or N237D;
compared with the amino acid sequence as set forth in SEQ ID NO 18, an site mutation of the L protein comprises one or more of S87P orI487T.

2. The method for treating a tumor with a combination of a recombinant oncolytic virus and a macromolecular antibody-based anti-cancer drug according to claim 1, **characterized in that** the recombinant oncolytic virus comprises one or more of rhabdovirus, poxvirus, herpes simplex virus, measles virus, Semliki Forest virus, poliomyelitis virus, reovirus, Seneca Valley virus, echovirus, Coxsackie virus, Newcastle disease virus, or Maraba virus.

3. The method for treating a tumor with a combination of a recombinant oncolytic virus and a macromolecular antibody-based anti-cancer drug according to claim 2, **characterized in that** the rhabdovirus comprises a vesicular stomatitis virus.

4. The method for treating a tumor with a combination of a recombinant oncolytic virus and a macromolecular antibody-based anti-cancer drug according to claim 1, **characterized in that**:
the M protein comprises an amino acid sequence as set forth in any one of SEQ ID NOs 4-11;
the G protein comprises an amino acid sequence as set forth in SEQ ID NO 13;
the N protein comprises an amino acid sequence as set forth in SEQ ID NO 15;
the P protein comprises an amino acid sequence as set forth in SEQ ID NO 17; and
the L protein comprises an amino acid sequence as set forth in SEQ ID NO 19.

5. The method for treating a tumor with a combination of a recombinant oncolytic virus and a macromolecular antibody-based anti-cancer drug according to claim 1, **characterized in that**:
a method for administering the recombinant oncolytic virus or the macromolecular antibody-based anti-cancer drug comprises one or more of intratumoral injection, intravenous injection, intraperitoneal injection, intrathoracic injection, pelvic injection, subcutaneous injection, intrathecal injection, intramuscular injection, or intranasal administration.

6. The method for treating a tumor with a combination of a recombinant oncolytic virus and a macromolecular antibody-based anti-cancer drug according to claim 1, **characterized in that** the recombinant oncolytic virus further comprises an antigen encoded by an exogenous gene.

7. The method for treating a tumor with a combination of a recombinant oncolytic virus and a macromolecular antibody-based anti-cancer drug according to claim 1, **characterized in that** the antigen is selected from a hematological tumor antigen and a solid tumor antigen.

8. The method for treating a tumor with a combination of a recombinant oncolytic virus and a macromolecular antibody-based anti-cancer drug according to claim 7, **characterized in that**:
the solid tumor antigen comprises 5T4, RORl, EGFR, FcγRI, FcγRIIa, FcγRIIb, CD24, CD28, CD137, CTLA-4, HER2, HER3, FAS, FAP, LGR5, C5aR1, A2AR, FGFR1, FGFR2, FGFR3, FGFR4, glucocorticoid-induced TNFR-related protein, LTβR, TRAIL receptor 1, TRAIL receptor 2, prostate specific membrane antigen protein (PSMA), prostate stem cell antigen protein, tumor-associated protein carbonic anhydrase IX, EGFR1, EGFRvIII, ErbB3, a folate receptor, an ephrin receptor, PDGFRa, ErbB-2, CD2, CD40, CD74, CD80, CD86, CCAM5, CCAM6, p53, MET, HGFR, MAGE-A1, MAGE-A2, MAGE-A3, MAGE-A4, MAGE-A6, MAGE-A10, MAGE-A12, BACE, DAM-6, DAM-10, GAGE-1, GAGE-2, GAGE-8, GAGE-3, GAGE-4, GAGE-5, GAGE-6, GAGE-7B, NA88-A, NY-ESO-1, BRCA1, BRCA2, MART-1, MC1R, Gp100, PSA, PSM, tyrosinase, TRP-1, TRP-2, ART-4, CAMEL, Cyp-B, hTERT, hTRT, iCE, MUC2, P-cadherin, myostatin, Cripto, MUC5AC, PRAME, P15, RU1, RU2, SART-1, SART-3, AFP, β-catenin/m, caspase-8/m, CDK-4/m, ELF2M, GnT-V, G250, HSP70-2M, HST-2, KIAA0205, MUM-1, MUM-2, MUM-3, myosin/m, RAGE, SART-2, TRP-2/INT2, 707-AP, annexin II, CDC27/m, TPI/mbcr-abl, ETV6/AML, LDLR/FUT, Pml/RARα, TEL/AML1, CD28, CD137, CanAg, Mesothelin (MSLN), DR5, PD-1, PD-L1, IGF-1R, CXCR4, neuropilin 1, glypicans, EphA2, B7-H3, B7-H4, gpA33, GPC3, SSTR2, GD2, VEGF-A, VEGFR-2, PDGFR-a, ANKL, RANKL, MSLN, EBV, TROP2, FOLR1, AXL, Claudin 18.2, MUC1, TPBG, CEA, EpCAM, Nectin-4, KARS, CD39, CD73, TIGIT, CD47, DLL3, and Claudin 18.1; and
the hematological tumor antigen comprises BCMA, CD4, CD5, CD7, CD10, FcγRIIIa, FcγRIIIb, CD19, CD20, CD22, CD23, CD30, CD33, CD34, CD37, CD38, CD44, CD47, CD56, CD70, CD117, CD123, CD138, CD174, CLL-1, ROR1, NKG2DL1/2, IL1R3, FCRL5, GPRC5D, CLEC12A, WT1, FLT3, TLR8, SHP2, KAT6A/B, CSNK1A1, FLI1, IKZF1/3, PI3K, c-Kit, SLAMF3, SLAMF7, TCR B-chain, ITGB7, k-1gG, TACI, TRBCI, LeY, and CD79b.

9. The method for treating a tumor with a combination of a recombinant oncolytic virus and a macromolecular antibody-based anti-cancer drug according to claim 6, **characterized in that** the antigen expressed by the recombinant oncolytic virus is at least one or more antigens, each being a full-length or partial sequence thereof.

10. The method for treating a tumor with a combination of a recombinant oncolytic virus and a macromolecular antibody-based anti-cancer drug according to claim 1, **characterized in that** an amino acid sequence of the antigen expressed by the recombinant oncolytic virus is one or more of:
antigen HER2 comprising an amino acid sequence as set forth in SEQ ID NO 20;
antigen EGFR comprising an amino acid sequence as set forth in SEQ ID NO 21;
antigen 5T4 (TPBG) comprising an amino acid sequence as set forth in SEQ ID NO 22;
antigen B7-H3 comprising an amino acid sequence as set forth in SEQ ID NO 23;
antigen TROP2 comprising an amino acid sequence as set forth in SEQ ID NO 24;
antigen BCMA comprising an amino acid sequence as set forth in SEQ ID NO 25;
antigen VEGFR-2 comprising an amino acid sequence as set forth in SEQ ID NO 26;
antigen SLAMF7 comprising an amino acid sequence as set forth in SEQ ID NO 27;
antigen CD19 comprising an amino acid sequence as set forth in SEQ ID NO 28;
antigen CD19 comprising an amino acid sequence as set forth in SEQ ID NO 29;
antigen CD79b comprising an amino acid sequence as set forth in SEQ ID NO 30;
antigen GPC3 comprising an amino acid sequence as set forth in SEQ ID NO 31;
antigen GPC3 comprising an amino acid sequence as set forth in SEQ ID NO 32;
antigen GPC3 comprising an amino acid sequence as set forth in SEQ ID NO 33;
antigen Claudin 18.2 comprising an amino acid sequence as set forth in SEQ ID NO 34;
antigen Claudin 18.2 comprising an amino acid sequence as set forth in SEQ ID NO 35;
antigen VEGF comprising an amino acid sequence as set forth in SEQ ID NO 36;
antigen HER3 comprising an amino acid sequence as set forth in SEQ ID NO 37;
antigen NY-ESO-1 comprising an amino acid sequence as set forth in SEQ ID NO 38;
antigen MLSN comprising an amino acid sequence as set forth in SEQ ID NO 39;
antigen CD30 comprising an amino acid sequence as set forth in SEQ ID NO 40;
antigen MUC1 comprising an amino acid sequence as set forth in SEQ ID NO 41;
antigen MAGE A4 comprising an amino acid sequence as set forth in SEQ ID NO 42;
antigen MAGE A4 comprising an amino acid sequence as set forth in SEQ ID NO 43;
antigen Met comprising an amino acid sequence as set forth in SEQ ID NO 44; or
antigen CD22 comprising an amino acid sequence as set forth in SEQ ID NO 45.

11. The method for treating a tumor with a combination of a recombinant oncolytic virus and a macromolecular antibody-based anti-cancer drug according to claim 1, **characterized in that** the macromolecular antibody-based anti-cancer drug comprises:
a macromolecular antibody-based anti-cancer drug targeting HER2, comprising Trastuzumab, Pertuzumab, Ado-trastuzumab emtansine, Disitamab, Margetuximab, Inetetamab, Fam-trastuzumab deruxtecan-nxki, Trastuzumab deruxtecan, and Disitamab vedotin;
a macromolecular antibody-based anti-cancer drug targeting EGFR, comprising Cetuximab, Panitumumab, Necitumumab, Nimotuzumab, Amivantamab, and Getuximab Saratolacan;
a macromolecular antibody-based anti-cancer drug targeting PD-1, comprising Nivolumab, Pembrolizumab, Tislelizumab, Sintilimab, Cemiplimab, Toripalimab, Camrelizumab, Dostarlimab, Penpulimab, Zimberelimab, and Cadonilimab;
a macromolecular antibody-based anti-cancer drug targeting PD-L1, comprising Atezolizumab, Durvalumab, Avelumab, and Sugemalimab;
a macromolecular antibody-based anti-cancer drug targeting TROP2, comprising Sacituzumab govitecan;
a macromolecular antibody-based anti-cancer drug targeting BCMA, comprising Belantamab Mafodotin, Elranatamab, and Teclistamab;
a macromolecular antibody-based anti-cancer drug targeting VEGFR-2, comprising Ramucirumab;
a macromolecular antibody-based anti-cancer drug targeting SLAMF7, comprising Elotuzumab;
a macromolecular antibody-based anti-cancer drug targeting CD3, comprising Blinatumomab, Epcoritamab, Glofitamab, and Mosunetuzumab;
a macromolecular antibody-based anti-cancer drug targeting CD19, comprising Blinatumomab and Loncastuximab tesirine;
a macromolecular antibody-based anti-cancer drug targeting CD20, comprising Rituximab, Obinutuzumab, Tosituomab, Ofatumumab, Ibritumomab, and Ibritumomab tiuxetan;
a macromolecular antibody-based anti-cancer drug targeting CD22, comprising Inotuzumab Ozogamicin and Moxetumomab Pasudotox;
a macromolecular antibody-based anti-cancer drug targeting CD30, comprising Brentuximab;
a macromolecular antibody-based anti-cancer drug targeting CD33, comprising Gemtuzumab and Gemtuzumab ozogamicin;
a macromolecular antibody-based anti-cancer drug targeting CD38, comprising Daratumumab;
a macromolecular antibody-based anti-cancer drug targeting CD52, comprising Alemtuzumab;
a macromolecular antibody-based anti-cancer drug targeting CD79b, comprising Polatuzumab vedotin;
a macromolecular antibody-based anti-cancer drug targeting Met, comprising Amivantamab;
a macromolecular antibody-based anti-cancer drug targeting GD2, comprising Dinutuximab;
a macromolecular antibody-based anti-cancer drug targeting VEGF, comprising Bevacizumab and Byvasda;
a macromolecular antibody-based anti-cancer drug targeting PDGFR-α, comprising Olaratumab;
a macromolecular antibody-based anti-cancer drug targeting CTLA-4, comprising Ipilimumab;
a macromolecular antibody-based anti-cancer drug targeting RANKL, comprising Denosumab;
a macromolecular antibody-based anti-cancer drug targeting FRa, comprising Mirvetuximab and Mirvetuximab Soravtansine;
a macromolecular antibody-based anti-cancer drug targeting TF, comprising Tisotumab vedotin;
a macromolecular antibody-based anti-cancer drug targeting IL6, comprising Siltuximab;
a macromolecular antibody-based anti-cancer drug targeting GPRC5D, comprising Talquetamab;
a macromolecular antibody-based anti-cancer drug targeting TNF-α, comprising Ozoralizumab;
a macromolecular antibody-based anti-cancer drug targeting EPCAM, comprising Catumaxomab;
a macromolecular antibody-based anti-cancer drug targeting 5T4, comprising Anti-5T4 antibody [EPR5529] (ab134162), GEN-1044, ALG.APV-527, SYD-1875, ASN-004, CBA-1535, and DM004;
a macromolecular antibody-based anti-cancer drug targeting B7-H3, comprising Anti-CD276 antibody [EPNCIR122] (ab134161), Omburtamab, Enoblituzumab, Omburtamab, MGC018, Obrindatamab, Mirzotamab clezutoclax, TAK 280, YBL 018, ATG 027, MIL108, ITC 6146RO, GTB 5550, Hu8H9, SHR-1812, XmAb 808, BAT 8009, and HS-20093;
a macromolecular antibody-based anti-cancer drug targeting GPC3, comprising Anti-Glypican 3 antibody [SP86] (ab95363), ECT-204, Anti-CD3/MUC1-armed-cytokine induced killer cells, Codrituzumab, GPC- 3298306, ERY-974, MDX-1414, B010-A, HLX-63, and LQ-102; and
a macromolecular antibody-based anti-cancer drug targeting Claudin 18.2, comprising Anti-Claudin18.2 antibody [EPR19202] (ab222512), Zolbetuximab, Q-1802, LM-102, TJ-CD48, Recombinant humanized anti-Claudin 18.2 monoclonal antibody, HBM-1029, HLX-58, and SOT-102.

12. The method for treating a tumor with a combination of a recombinant oncolytic virus and a macromolecular antibody-based anti-cancer drug according to claim 1, **characterized in that** the macromolecular antibody-based anti-cancer drug is one or more selected from a group consisting of: Trastuzumab, Necitumumab, Pembrolizumab, Atezolizumab, Sacituzumab govitecan, Belantamab Mafodotin, Ramucirumab, Elotuzumab, Blinatumomab, Loncastuximab tesirine, Polatuzumab vedotin, Anti-5T4 antibody [EPR5529] (ab134162), Anti-CD276 antibody [EPNCIR122] (ab134161), Anti-Glypican 3 antibody [SP86] (ab95363), and Anti-Claudin18.2 antibody [EPR19202] (ab222512).

13. The method for treating a tumor with a combination of a recombinant oncolytic virus and a macromolecular antibody-based anti-cancer drug according to claim 1, **characterized in that** the macromolecular antibody-based anti-cancer drug comprises a monoclonal antibody-based drug, a polyclonal antibody-based drug, and an antibody-drug conjugate, each targeting a specific target.

14. The method for treating a tumor with a combination of a recombinant oncolytic virus and a macromolecular antibody-based anti-cancer drug according to claim 1, **characterized in that** the recombinant oncolytic virus further comprises a cytokine encoded by an exogenous gene.

15. The method for treating a tumor with a combination of a recombinant oncolytic virus and a macromolecular antibody-based anti-cancer drug according to claim 14, **characterized in that** the cytokine is selected from interleukins, interferons, tumor necrosis factors, colony stimulating factors, transforming growth factor β, and the chemokine family.

16. The method for treating a tumor with a combination of a recombinant oncolytic virus and a macromolecular antibody-based anti-cancer drug according to claim 14, **characterized in that** the cytokine is one or more selected from a group consisting of : GMCSF, G-CSF, M-CSF, IL-1, IL-2, IL-4, IL-5, IL-6, IL-9, IL-10, IL-12, IL-13, IL-15, IL-17, IL-18, IL-23, IL-27, IFN-α, IFN-β, IFN-γ, IFN-β, TGF-β, and TNF-α.

17. The method for treating a tumor with a combination of a recombinant oncolytic virus and a macromolecular antibody-based anti-cancer drug according to claim 14, **characterized in that** an amino acid sequence of the cytokine is one or more of:
cytokine IL-2 comprising an amino acid sequence as set forth in SEQ ID NO 46;
cytokine IL-2 comprising an amino acid sequence as set forth in SEQ ID NO 47;
cytokine IL-12A comprising an amino acid sequence as set forth in SEQ ID NO 48;
cytokine IL-12B comprising an amino acid sequence as set forth in SEQ ID NO 49;
cytokine GMCSF comprising an amino acid sequence as set forth in SEQ ID NO 50;
cytokine IL-2 comprising an amino acid sequence as set forth in SEQ ID NO 51;
cytokine IL-15 comprising an amino acid sequence as set forth in SEQ ID NO 52;
cytokine IL-15 comprising an amino acid sequence as set forth in SEQ ID NO 53;
cytokine IL-18 comprising an amino acid sequence as set forth in SEQ ID NO 54;
cytokine IL-18 comprising an amino acid sequence as set forth in SEQ ID NO 55;
cytokine IFN-α comprising an amino acid sequence as set forth in SEQ ID NO 56;
cytokine IFN-β comprising an amino acid sequence as set forth in SEQ ID NO 57;
cytokine IL-12A comprising an amino acid sequence as set forth in SEQ ID NO 58;
cytokine IL-12A comprising an amino acid sequence as set forth in SEQ ID NO 59; or
cytokine IL-12A comprising an amino acid sequence as set forth in SEQ ID NO 60.

18. The method for treating a tumor with a combination of a recombinant oncolytic virus and a macromolecular antibody-based anti-cancer drug according to claim 1, **characterized in that** the recombinant oncolytic virus comprises a nucleic acid molecule; and the nucleic acid molecule comprises a nucleic acid sequence encoding the M protein with the site mutation, a nucleic acid sequence encoding the G protein with the site mutation, a nucleic acid sequence encoding the N protein with thesite mutation, a nucleic acid sequence encoding the P protein with the site mutation, and a nucleic acid sequence encoding the L protein with the site mutation.

19. The method for treating a tumor with a combination of a recombinant oncolytic virus and a macromolecular antibody-based anti-cancer drug according to claim 18, **characterized in that** the nucleic acid molecule further comprises at least one of a nucleic acid sequence encoding a cytokine, or a nucleic acid sequence encoding an antigen.

20. The method for treating a tumor with a combination of a recombinant oncolytic virus and a macromolecular antibody-based anti-cancer drug according to claim 19, **characterized in that** the nucleic acid sequence encoding the cytokine is located between the nucleic acid sequence encoding the G protein with the site mutation and the nucleic acid sequence encoding the L protein with the site mutation; and/or, the nucleic acid sequence encoding an antigen is located between the nucleic acid sequence encoding the G protein with the site mutation and the nucleic acid sequence encoding the L protein with the site mutation.

21. The method for treating a tumor with a combination of a recombinant oncolytic virus and a macromolecular antibody-based anti-cancer drug according to claim 1, **characterized in that** the method for treating a tumor with a combination of the recombinant oncolytic virus and the macromolecular antibody-based anti-cancer drug is used for continuously killing abnormally proliferative cells.

22. The method for treating a tumor with a combination of a recombinant oncolytic virus and a macromolecular antibody-based anti-cancer drug according to claim 21, **characterized in that** the abnormally proliferative cells are selected from tumor cells or tumor tissue-related cells.

23. The method for treating a tumor with a combination of a recombinant oncolytic virus and a macromolecular antibody-based anti-cancer drug according to claim 22, **characterized in that** the tumor comprises a solid tumor or a hematological tumor.

24. The method for treating a tumor with a combination of a recombinant oncolytic virus and a macromolecular antibody-based anti-cancer drug according to claim 22, **characterized in that** the tumor comprises acute lymphoblastic leukemia, acute B lymphocytic leukemia, chronic non-lymphocytic leukemia, non-Hodgkin lymphoma, anal cancer, astrocytoma, basal cell carcinoma, bile duct cancer, bladder cancer, mastocarcinoma, breast cancer, cervical cancer, chronic myeloproliferative neoplasm, colorectal cancer, endometrial carcinoma, ependymoma, esophageal cancer, diffuse large B cell lymphoma, esthesioneuroblastoma, Ewing's sarcoma, fallopian tube carcinoma, gallbladder carcinoma, gastric cancer, gastrointestinal carcinoid tumor, hepatocellular carcinoma, hypopharyngeal carcinoma, Kaposi's sarcoma, kidney cancer, Langerhans cell histiocytosis, laryngeal cancer, liver cancer, lung cancer, melanoma, Merkel cell carcinoma, mesothelioma, oral cancer, neuroblastoma, non-small cell lung cancer, osteosarcoma, ovarian cancer, pancreatic cancer, pancreatic neuroendocrine tumor, pharyngeal carcinoma, pituitary adenoma, prostate cancer, rectal cancer, renal cell carcinoma, retinoblastoma, skin cancer, small cell lung cancer, small intestine cancer, neck squamous cell carcinoma, testicular cancer, thymoma, thyroid cancer, uterine cancer, vaginal cancer, and hemangioma.

25. The method for treating a tumor with a combination of a recombinant oncolytic virus and a macromolecular antibody-based anti-cancer drug according to claim 22, **characterized in that** through expressing a tumor antigen target with the recombinant oncolytic virus and administering the macromolecular antibody-based anti-cancer drug targeting the tumor antigen target in combination to attack and kill the tumor cells, thereby achieving a therapeutic effect comparable or superior to that of a monotherapy of the recombinant oncolytic virus or a monotherapy of the macromolecular antibody-based anti-cancer drug.

26. A composition, **characterized by** comprising the recombinant oncolytic virus and the macromolecular antibody-based anti-cancer drug in the method according to claim 1, and the macromolecular antibody-based anti-cancer drug comprises a macromolecular antibody-based anti-cancer drug targeting HER2, a macromolecular antibody-based anti-cancer drug targeting EGFR, a macromolecular antibody-based anti-cancer drug targeting PD-1, a macromolecular antibody-based anti-cancer drug targeting PD-L1, a macromolecular antibody-based anti-cancer drug targeting TROP2, a macromolecular antibody-based anti-cancer drug targeting BCMA, a macromolecular antibody-based anti-cancer drug targeting VEGFR-2, a macromolecular antibody-based anti-cancer drug targeting SLAMF7, a macromolecular antibody-based anti-cancer drug targeting CD3, a macromolecular antibody-based anti-cancer drug targeting CD19, a macromolecular antibody-based anti-cancer drug targeting CD20, a macromolecular antibody-based anti-cancer drug targeting CD22, a macromolecular antibody-based anti-cancer drug targeting CD30, a macromolecular antibody-based anti-cancer drug targeting CD33, a macromolecular antibody-based anti-cancer drug targeting CD38, a macromolecular antibody-based anti-cancer drug targeting CD52, a macromolecular antibody-based anti-cancer drug targeting CD79b, a macromolecular antibody-based anti-cancer drug targeting Met, a macromolecular antibody-based anti-cancer drug targeting GD2, a macromolecular antibody-based anti-cancer drug targeting VEGF, a macromolecular antibody-based anti-cancer drug targeting PDGFR-α, a macromolecular antibody-based anti-cancer drug targeting CTLA-4, a macromolecular antibody-based anti-cancer drug targeting RANKL, a macromolecular antibody-based anti-cancer drug targeting FRa, a macromolecular antibody-based anti-cancer drug targeting TF, a macromolecular antibody-based anti-cancer drug targeting IL6, a macromolecular antibody-based anti-cancer drug targeting GPRC5D, a macromolecular antibody-based anti-cancer drug targeting TNF-α, a macromolecular antibody-based anti-cancer drug targeting EPCAM, a macromolecular antibody-based anti-cancer drug targeting CD24, a macromolecular antibody-based anti-cancer drug targeting 5T4, a macromolecular antibody-based anti-cancer drug targeting B7-H3, a macromolecular antibody-based anti-cancer drug targeting GPC3, and a macromolecular antibody-based anti-cancer drug targeting Claudin 18.2;
the recombinant oncolytic virus comprises an M protein, a G protein, an N protein, a P protein, and an L protein;
compared with an amino acid sequence as set forth in SEQ ID NO 1,
an site mutation of the M protein comprises one or more of M51R, V221F, or S226R;
alternatively, the site mutation of the M protein comprises one or more of N32S, N49D, M51R, H54Y, V221F, V225I, or S226R;
alternatively, the site mutation of the M protein comprises one or more of N32S, N49D, M51R, H54Y, knocking out of nucleotide bases encoding leucine at position 111, V221F, V225I, or S226R;
alternatively, the site mutation of the M protein comprises one or more of N32S, N49D, M51R, H54Y, L111A, V221F, V225I, or S226R;
alternatively, the site mutation of the M protein comprises one or more of G21E, N32S, N49D, M51R, H54Y, V221F, V225I, or S226R;
alternatively, the site mutation of the M protein comprises one or more of G21E, N32S, M33A, N49D, M51R, H54Y, V221F, V225I, or S226R;
alternatively, the site mutation of the M protein comprises one or more of G21E, N32S, M33A, N49D, M51R, H54Y, A133T, V221F, V225I, or S226R;
alternatively, the site mutation of the M protein comprises one or more of N32S, M33A, N49D, M51R, H54Y, V221F, V225I, or S226R;
alternatively, the site mutation of the M protein comprises one or more of N32S, M33A, N49D, M51R, H54Y, A133T, V221F, V225I, or S226R;
alternatively, the site mutation of the M protein comprises one or more of N32S, N49D, M51R, H54Y, A133T, V221F, V225I, or S226R;
compared with an amino acid sequence as set forth in SEQ ID NO 12, an site mutation of the G protein comprises one or more of V53I, A141V, D172Y, K217E, D232G, V331A, V371E, G436D, T438S, F453L, T471I, or Y487H;
compared with an amino acid sequence as set forth in SEQ ID NO 14, an site mutation of the N protein comprises one or more of I14V, R155K, or S353N;
compared with an amino acid sequence as set forth in SEQ ID NO 16, an site mutation of the P protein comprises one or more of R50K, V76A, D99E, L126S, L140S, H151Y, I168M, K170E, Y189S, or N237D;
compared with an amino acid sequence as set forth in SEQ ID NO 18, an site mutation of the L protein comprises one or more of S87P or I487T.
